# EUROPEAN PATENT APPLICATION

(11) **EP 4 509 045 A1**
(43) Date of publication of application: **19.02.2025**
(21) Application number: 22937064.8
(22) Date of filing: 07.06.2022
(51) Int. Cl.: A61B 5/145

(54) **BLOOD GLUCOSE PREDICTION METHOD AND DEVICE COMBINING BIG DATA MODEL AND PERSONALIZED MODEL**

(30) Priority: 12.04.2022 CN 202210380666
(71) Applicant: Lepu Medical Technology (Beijing) Co., Ltd., Beijing 102200 (CN)
(72) Inventor: ZHANG, Biying, Beijing 102200 (CN); ZHANG, Hongpan, Beijing 102200 (CN); LI, Ruilai, Beijing 102200 (CN); CAO, Jun, Beijing 102200 (CN)
(74) Representative: Glück Kritzenberger Patentanwälte PartGmbB
(86) International application number: PCT/CN2022/097249
(87) International publication number: WO 2023/197426

(57) **Abstract**

A blood glucose prediction method and device combining a big data model and a personalized model. The method comprises: training a big data blood glucose prediction model; receiving a first collection data set of a specified object; when a first data type is a label type, according to the first collection data set, updating a personalized data base; if the updating is successful, counting the total number of personalized data records to generate a first total number; if the first total number is greater than or equal to a first threshold value, screening personalized data calibrated records; if the first total number is equal to a second threshold value, training a personalized blood glucose prediction model; when the first data type is a prediction type, counting the total number of personalized data records to generate a second total number; if the second total number is smaller than the second threshold value, on the basis of the big data blood glucose prediction model, making a prediction; and if the second total number is equal to the second threshold value, on the basis of the big data blood glucose prediction model and the personalized blood glucose prediction model, making a personalized blood glucose prediction. The method can avoid the inconvenience of invasive blood glucose tests.

## Description

This application claims the priority of the Chinese patent application filed with the China National Intellectual Property Administration on April 12, 2022 with the application number 202210380666.9 and the tile "BLOOD GLUCOSE PREDICTION METHOD AND DEVICE COMBINING BIG DATA MODEL AND PERSONALIZED MODEL".

### BACKGROUND OF THE INVENTION

### 1. Technical Field

The present invention relates to the technical field of data processing, in particular to a blood glucose prediction method and device combining a big data model and a personalized model.

### 2. Description of Related Art

The glucose in the blood is referred to as blood glucose (Glu). Blood glucose needs to be maintained at a certain level to ensure the normal functioning of the body's organs and tissue. The prevalent method for blood glucose monitoring involves extracting blood from the body through invasive means and conducting chemical analysis on the extracted blood to obtain the blood glucose measurement. Due to the trauma caused to the body by this method, long-term blood glucose monitoring using this approach would undoubtedly bring a lot of inconvenience to users.

Based on the Beer-Lambert law, we know that the absorption of transmitted light by a solution is related to the concentration of the solute. Therefore, we can determine that higher concentrations of glucose in the blood result in lower transmission light intensity through human tissue. Thus, by collecting photoplethysmography (PPG) signals that reflect changes in blood light transmission intensity, blood glucose changes can be observed.

Based on the Metabolic Heat Conformation (MHC) theory, we know that the body's energy rhythms in different time periods are somewhat related to the energy released after metabolism. The oxidation of glucose and subsequent energy production can be dispersed as heat from the body to the environment, thus correlating body metabolic heat with glucose levels and oxygen supply. That is, under the condition of adequate oxygen supply, body metabolic heat can be utilized as a reference for monitoring changes in blood glucose levels based on the MHC theory.

### BRIEF SUMMARY OF THE INVENTION

The purpose of the present invention is to provide a blood glucose prediction method and device combining a big data model and a personalized model, electronic equipment and a computer-readable storage medium to overcome the shortcomings of the prior art. A big data blood glucose prediction model and a personalized blood glucose prediction model are created in advance based on the correlation between optics, metabolic heat and blood glucose; in the case of insufficient precipitation of personalized data, blood glucose prediction is performed based on the big data blood glucose prediction model; and under the condition of sufficient personalized data precipitation, a corresponding personalized blood glucose prediction model is trained for each specified object, and personalized blood glucose prediction is performed for each object based on the big data blood glucose prediction model and the personalized blood glucose prediction model. Through the present invention, not only can the inconvenience caused by invasive blood glucose tests be solved, but also different prediction model schemes can be provided for different objects, thus achieving the purposes of reducing observation difficulty and improving the level of prediction customization.

To achieve the above purposes, a first aspect of the embodiments of the present invention provides a blood glucose prediction method combining a big data model and a personalized model. The method comprises:
training a big data blood glucose prediction model based on a preset big data base and confirming a big data calibrated record;
receiving a first collection data set of a specified object, the first collection data set comprising a first data type, and the first data type being a prediction type or a label type;
when the first data type is a label type, updating a personalized data base corresponding to the specified object according to the first collection data set; if the data base is successfully updated, counting the total number of personalized data records of the personalized data base to generate a corresponding first total number; if the first total number is greater than or equal to a preset first threshold value, performing optimal personalized data calibrated record screening in the personalized data base based on the big data blood glucose prediction model to obtain a latest personalized data calibrated record; if the first total number is equal to a preset second threshold value, training a personalized blood glucose prediction model based on the big data blood glucose prediction model, the personalized data base and the personalized data calibrated record; the first threshold value being smaller than the second threshold value;
when the first data type is a prediction type, counting the total number of personalized data records in the personalized data base to generate a corresponding second total number; if the second total number is smaller than the second threshold value, performing blood glucose prediction processing on the first collection data set based on the second total number, the big data calibrated record, the personalized data calibrated record and the big data blood glucose prediction model to generate corresponding first predicted blood glucose data; and if the second total number is equal to the second threshold value, performing personalized blood glucose prediction processing on the first collection data set based on the personalized data calibrated record, the big data blood glucose prediction model and the personalized blood glucose prediction model to generate corresponding second predicted blood glucose data.

Optionally, a neural network of the big data blood glucose prediction model adopts a fully connected network structure; and
a model structure of the personalized blood glucose prediction model is a support vector machine model structure, a random forest model structure or a decision tree model structure.

Optionally, the big data base comprises a plurality of big data records; the big data records comprise first preprandial sub-records and first postprandial sub-records; the first preprandial sub-record comprises a first collection time, a first time interval, a first optical data group, a first metabolic heat data group, a first physiological information data group and a first measured blood glucose level; the first postprandial sub-record comprises a second collection time, a second time interval, a second optical data group, a second metabolic heat data group, a second physiological information data group and a second measured blood glucose level; the first and second measured blood glucose levels correspond to preprandial and postprandial measured blood glucose levels respectively;
when the first data type is a label type, the first collection data set further comprises a first preprandial collection record and a first postprandial collection record; the first preprandial collection record comprises a third collection time, a third time interval, a third optical data group, a third metabolic heat data group, a third physiological information data group and a third measured blood glucose level; the first postprandial collection record comprises a fourth collection time, a fourth time interval, a fourth optical data group, a fourth metabolic heat data group, a fourth physiological information data group and a fourth measured blood glucose level; the third and fourth measured blood glucose levels correspond to preprandial and postprandial measured blood glucose levels respectively;
when the first data type is a prediction type, the first collection data set further comprises a fifth collection time, a fifth time interval, a fifth optical data group, a fifth metabolic heat data group and a fifth physiological information data group;
the personalized data base comprises the plurality of personalized data records; the personalized data records comprise second preprandial sub-records and second postprandial sub-records; the second preprandial sub-record comprises a sixth collection time, a sixth time interval, a sixth optical data group, a sixth metabolic heat data group, a sixth physiological information data group and a fifth measured blood glucose level; the second postprandial sub-record comprises a seventh collection time, a seventh time interval, a seventh optical data group, a seventh metabolic heat data group, a seventh physiological information data group and a sixth measured blood glucose level; the fifth and sixth measured blood glucose levels correspond to preprandial and postprandial measured blood glucose levels respectively; and the maximum total number of records in the personalized data base is the second threshold value.

Optionally, the step of training a big data blood glucose prediction model based on a preset big data base and confirming a big data calibrated record specifically comprises:
extracting the plurality of big data records from the big data base to form a big data set;
performing first feature vector conversion processing on each of the first preprandial sub-records or the first postprandial sub-records in the large data set to generate first feature vectors corresponding to each sub-record; recording the first measured blood glucose levels or the second measured blood glucose levels corresponding to each of the first feature vectors as corresponding first feature blood glucose levels;
selecting a big data record from the big data set as the big data calibrated record; taking two first feature vectors of the big data calibrated record as a corresponding big data preprandial calibration vector and a big data postprandial calibration vector respectively, and taking two first feature blood glucose levels as a corresponding big data preprandial calibrated blood glucose level and a big data postprandial calibrated blood glucose level respectively;
recording all the other first feature vectors except the big data preprandial calibration vector and the big data postprandial calibration vector as second feature vectors; forming corresponding first model training vectors by each of the second feature vectors, the big data preprandial calibration vector, the big data postprandial calibration vector, the big data preprandial calibrated blood glucose level and the big data postprandial calibrated blood glucose level;
based on the big data blood glucose prediction model, performing big data model prediction processing on any of the first model training vectors to obtain corresponding first predicted data, and generating corresponding current predicted blood glucose data according to the sum of the big data postprandial calibrated blood glucose level and the first predicted data; calculating a training error of the current predicted blood glucose data in comparison with the first feature blood glucose level corresponding to the current first model training vector using a loss function of the big data blood glucose prediction model; when the training error does not meet a preset reasonable error range, modulating the big data blood glucose prediction model based on the training error, and selecting the next first model training vector to continue training the modulated big data blood glucose prediction model; and stopping training when the training error meets the reasonable error range.

Optionally, the step of updating a personalized data base corresponding to the specified object according to the first collection data set specifically comprises:
counting the total number of personalized data records in the personalized data base to generate a corresponding current total number; when the current total number is smaller than the second threshold value, adding a new personalized data record as a current record in the personalized data base; when the current total number is equal to the second threshold value, taking the personalized data record with the earliest addition time in the personalized data base as the current record; and
extracting the first preprandial collection record from the first collection data set as the second preprandial sub-record of the current record and storing the same in the personalized data base, and extracting the first postprandial collection record as the second postprandial sub-record of the current record and storing the same in the personalized data base.

Optionally, the step of performing optimal personalized data calibrated record screening in the personalized data base based on the big data blood glucose prediction model to obtain a latest personalized data calibrated record specifically comprises:
performing first feature vector conversion processing on the second preprandial sub-record or the second postprandial sub-record of each of the personalized data records in the personalized data base to generate third feature vectors corresponding to each sub-record; recording the fifth measured blood glucose levels or the sixth measured blood glucose levels corresponding to each of the third feature vectors as corresponding second feature blood glucose levels;
sequentially extracting the personalized data records as a current calibrated record in the personalized data base; taking two third feature vectors of the current calibrated record as a corresponding big data preprandial calibration vector and a big data postprandial calibration vector respectively, and taking two second feature blood glucose levels as a corresponding big data preprandial calibrated blood glucose level and a big data postprandial calibrated blood glucose level respectively; recording third feature vectors of other personalized data records except the current calibrated record as fourth feature vectors, and forming corresponding first model input vectors by each of the fourth feature vectors, the big data preprandial calibration vector, the big data postprandial calibration vector, the big data preprandial calibrated blood glucose level and the big data postprandial calibrated blood glucose level; based on the big data blood glucose prediction model, performing big data model prediction processing on each of the first model input vectors to obtain corresponding second predicted data, and generating corresponding first model predicted blood glucose data according to the sum of the big data postprandial calibrated blood glucose level and the second predicted data; calculating an absolute difference between each of the first model predicted blood glucose data and the second feature blood glucose level corresponding to a current first model input vector to obtain a corresponding first prediction error; calculating the sum of all the obtained first prediction errors to obtain a first prediction error sum corresponding to the current calibrated record; and
taking a minimum prediction error sum as an optimal calibration personalized record group screening principle, and taking the personalized data record with the minimum first prediction error sum in the personalized data base as the latest personalized data calibrated record.

Optionally, the step of training a personalized blood glucose prediction model based on the big data blood glucose prediction model, the personalized data base and the personalized data calibrated record specifically comprises:
performing first feature vector conversion processing on the second preprandial sub-record or the second postprandial sub-record of each of the personalized data records in the personalized data base to generate fifth feature vectors corresponding to each sub-record; recording the fifth measured blood glucose levels or the sixth measured blood glucose levels corresponding to each of the fifth feature vectors as corresponding third feature blood glucose levels;
taking two fifth feature vectors of the personalized data calibrated record as a corresponding big data preprandial calibration vector and a big data postprandial calibration vector respectively, and taking two third feature blood glucose levels as a corresponding big data preprandial calibrated blood glucose level and a big data postprandial calibrated blood glucose level respectively;
recording the fifth feature vectors of other personalized data records except the personalized data calibrated record in the personalized data base as sixth feature vectors; forming corresponding second model training vectors by each of the sixth feature vectors, the big data preprandial calibration vector, the big data postprandial calibration vector, the big data preprandial calibrated blood glucose level and the big data postprandial calibrated blood glucose level;
performing big data model prediction processing on each of the second model training vectors based on the big data blood glucose prediction model to obtain corresponding third predicted data, and generating corresponding big data predicted blood glucose data according to the sum of the big data postprandial calibrated blood glucose level and the third predicted data;
according to a preset big data-personalized feature association status, extracting part of feature data from the sixth feature vectors corresponding to each of the second model training vectors to form corresponding seventh feature vectors, extracting part of feature data from the big data preprandial calibration vector to form a corresponding personalized preprandial calibration vector, and extracting part of feature data from the big data postprandial calibration vector to form a corresponding personalized postprandial calibration vector; forming corresponding third model training vectors by the seventh feature vectors, the personalized preprandial calibration vector, the personalized postprandial calibration vector, the big data preprandial calibrated blood glucose level, the big data postprandial calibrated blood glucose level and the big data predicted blood glucose data;
performing personalized model prediction processing on any of the third model training vectors based on the personalized blood glucose prediction model to obtain corresponding fourth predicted data, and generating corresponding current predicted blood glucose data according to the sum of the big data predicted blood glucose data and the fourth predicted data; calculating an absolute difference between the current predicted blood glucose data and the third feature blood glucose level corresponding to a current third model training vector as a current measurement error; modulating the personalized blood glucose prediction model when the current measurement error does not match a preset reasonable error range; and then selecting the next third model training vector to train the personalized blood glucose prediction model until training with the last third model training vector is completed.

Further, the step of, according to a preset big data-personalized feature association status, extracting part of feature data from the sixth feature vectors corresponding to each of the second model training vectors to form corresponding seventh feature vectors, extracting part of feature data from the big data preprandial calibration vector to form a corresponding personalized preprandial calibration vector, and extracting part of feature data from the big data postprandial calibration vector to form a corresponding personalized postprandial calibration vector, specifically comprises:
identifying whether each status bit of the big data-personalized feature association status is an association status, and if so, adding matching feature data of each status bit in the sixth feature vectors, the big data preprandial calibration vector and the big data postprandial calibration vector to the corresponding seventh feature vectors, the personalized preprandial calibration vector and the personalized postprandial calibration vector, the big data-personalized feature association status comprising first, second, third and fourth status bits, the matching feature data of the first status bit being time interval feature data, the matching feature data of the second status bit being optical feature sequences, the matching feature data of the third status bit being metabolic heat feature sequences, and the matching feature data of the fourth status bit being physiological feature sequences.

Optionally, the step of performing blood glucose prediction processing on the first collection data set based on the second total number, the big data calibrated record, the personalized data calibrated record and the big data blood glucose prediction model to generate corresponding first predicted blood glucose data specifically comprises:
determining whether the second total number is smaller than the first threshold value; if the second total number is smaller than the first threshold value, performing first feature vector conversion processing on the first preprandial sub-record and the first postprandial sub-record of the big data calibrated record, taking two obtained feature vectors as a corresponding big data preprandial calibration vector and a big data postprandial calibration vector, and taking the first measured blood glucose level and the second measured blood glucose level of the big data calibrated record as a corresponding big data preprandial calibrated blood glucose level and a big data postprandial calibrated blood glucose level; if the second total number is greater than or equal to the first threshold value, performing first feature vector conversion processing on the second preprandial sub-record and the second postprandial sub-record of the personalized data calibrated record, taking two obtained feature vectors as a corresponding big data preprandial calibration vector and a big data postprandial calibration vector, and taking the fifth measured blood glucose level and the sixth measured blood glucose level of the personalized data calibrated record as the corresponding big data preprandial calibrated blood glucose level and the big data postprandial calibrated blood glucose level;
performing first feature vector conversion processing on the first collection data set to generate corresponding eighth feature vectors; forming corresponding second model input vectors by the eighth feature vectors, the big data preprandial calibration vector, the big data postprandial calibration vector, the big data preprandial calibrated blood glucose level and the big data postprandial calibrated blood glucose level;
performing big data model prediction processing on the second model input vectors based on the big data blood glucose prediction model to obtain corresponding fifth predicted data; and generating the corresponding first predicted blood glucose data according to the sum of the big data postprandial calibrated blood glucose level and the fifth predicted data.

Optionally, the step of performing personalized blood glucose prediction processing on the first collection data set based on the personalized data calibrated record, the big data blood glucose prediction model and the personalized blood glucose prediction model to generate corresponding second predicted blood glucose data specifically comprises:
performing first feature vector conversion processing on the second preprandial sub-record and the second postprandial sub-record of the personalized data calibrated record to obtain two ninth feature vectors, taking the two ninth feature vectors as a corresponding big data preprandial calibration vector and a big data postprandial calibration vector respectively, and taking the fifth and sixth measured blood glucose levels of the personalized data calibrated record as a corresponding big data preprandial calibrated blood glucose level and a big data postprandial calibrated blood glucose level respectively;
performing first feature vector conversion processing on the first collection data set to generate corresponding tenth feature vectors; forming corresponding third model input vectors by the tenth feature vectors, the big data preprandial calibration vector, the big data postprandial calibration vector, the big data preprandial calibrated blood glucose level and the big data postprandial calibrated blood glucose level;
performing big data model prediction processing on the third model input vectors based on the big data blood glucose prediction model to obtain corresponding sixth predicted data; generating corresponding big data predicted blood glucose data according to the sum of the big data postprandial calibrated blood glucose level and the sixth predicted data;
according to a preset big data-personalized feature association status, extracting part of feature data from the tenth feature vectors to form corresponding eleventh feature vectors, extracting part of feature data from the big data preprandial calibration vector to form a corresponding personalized preprandial calibration vector, and extracting part of feature data from the big data postprandial calibration vector to form a corresponding personalized postprandial calibration vector;
forming corresponding fourth model input vectors by the eleventh feature vectors, the personalized preprandial calibration vector, the personalized postprandial calibration vector, the big data preprandial calibrated blood glucose level, the big data postprandial calibrated blood glucose level and the big data predicted blood glucose data;
performing personalized model prediction processing on the fourth model input vectors based on the personalized blood glucose prediction model to obtain corresponding seventh predicted data; and generating the corresponding second predicted blood glucose data according to the sum of the big data predicted blood glucose data and the seventh predicted data.

A second aspect of the embodiments of the invention provides a device for realizing the method described in the first aspect, comprising: a big data model preparation module, a data receiving module, a personalized model preparation module and a blood glucose prediction processing module;
the big data model preparation module is configured to train a big data blood glucose prediction model based on a preset big data base and confirm a big data calibrated record;
the data receiving module is configured to receive a first collection data set of a specified object, the first collection data set comprising a first data type, and the first data type being a prediction type or a label type;
the personalized model preparation module is configured to, when the first data type is a label type, update a personalized data base corresponding to the specified object according to the first collection data set; if the data base is successfully updated, count the total number of personalized data records of the personalized data base to generate a corresponding first total number; if the first total number is greater than or equal to a preset first threshold value, perform optimal personalized data calibrated record screening in the personalized data base based on the big data blood glucose prediction model to obtain a latest personalized data calibrated record; if the first total number is equal to a preset second threshold value, train a personalized blood glucose prediction model based on the big data blood glucose prediction model, the personalized data base and the personalized data calibrated record; the first threshold value being smaller than the second threshold value; and
the blood glucose prediction processing module is configured to, when the first data type is a prediction type, count the total number of personalized data records in the personalized data base to generate a corresponding second total number; if the second total number is smaller than the second threshold value, perform blood glucose prediction processing on the first collection data set based on the second total number, the big data calibrated record, the personalized data calibrated record and the big data blood glucose prediction model to generate corresponding first predicted blood glucose data; and if the second total number is equal to the second threshold value, perform personalized blood glucose prediction processing on the first collection data set based on the personalized data calibrated record, the big data blood glucose prediction model and the personalized blood glucose prediction model to generate corresponding second predicted blood glucose data.

A third aspect of the embodiments of the invention provides electronic equipments, comprising a memory, a processor and a transceiver;
the processor is configured to be coupled with the memory, and read and execute instructions in the memory, so as to realize the method described in the first aspect,
the transceiver is coupled with the processor, and the processor controls the transceiver to send and receive messages.

A fourth aspect of the embodiments of the invention provides a computer-readable storage medium, which stores computer instructions that, when executed by a computer, cause the computer to execute the method described in the first aspect.

The embodiments of the present invention provide a blood glucose prediction method and device combining a big data model and a personalized model, electronic equipment and a computer-readable storage medium. A big data blood glucose prediction model reflecting the correlation between optics, metabolic heat and blood glucose level changes is trained first, and a data record is selected from a big data base as a calibrated record of the model. A personalized data base is created for a specified object to store label data of a current object, that is, personalized records; after a sufficient number of personalized records are stored in the personalized data base, a personalized calibrated record more suitable for the specified object is selected from the personalized data base based on the well-trained big data blood glucose prediction model; and when the number of the personalized records reaches a specified threshold value, a personalized blood glucose prediction model is trained based on the well-trained big data blood glucose prediction model, the personalized calibrated record and the personalized data base. During blood glucose prediction of the specified object, if the total number of the personalized records has not reached the specified threshold value, the big data blood glucose prediction model is used for blood glucose prediction of the specified object; and if the total number of the personalized records reaches the specified threshold value, blood glucose prediction is performed on the specific object based on the big data blood glucose prediction model and the personalized blood glucose prediction model. By means of the present invention, in the case of insufficient precipitation of personalized data, blood glucose prediction is performed based on the big data blood glucose prediction model; and under the condition of sufficient personalized data precipitation, a corresponding personalized blood glucose prediction model is trained for each specified object, and personalized blood glucose prediction is performed for each object based on the big data blood glucose prediction model and the personalized blood glucose prediction model. In this way, not only can the inconvenience caused by invasive blood glucose tests be solved, but also different prediction model schemes can be provided for different objects, thus achieving the purposes of reducing observation difficulty and improving the level of prediction customization.

### BRIEF DESCRIPTION OF THE SEVERAL VIEWS OF THE DRAWINGS

Fig. 1 is a schematic diagram of a blood glucose prediction method combining a big data model and a personalized model according to Embodiment 1 of the present invention;
Fig. 2 is a modular structure diagram of a blood glucose prediction device combining a big data model and a personalized model according to Embodiment 2 of the present invention; and
Fig. 3 is a structural diagram of electronic equipment according to Embodiment 3 of the present invention.

### DETAILED DESCRIPTION OF THE INVENTION

In order to make the purpose, the technical scheme and advantages of the present invention more clear, the invention will be described in more detail below in combination with attached drawings. Obviously, the described embodiments are only part of the embodiments of the present invention, not all of them. Based on the embodiments of the present invention, all other embodiments obtained by those of ordinary skill in the art without making creative labor shall belong to the scope of protection of the present invention.

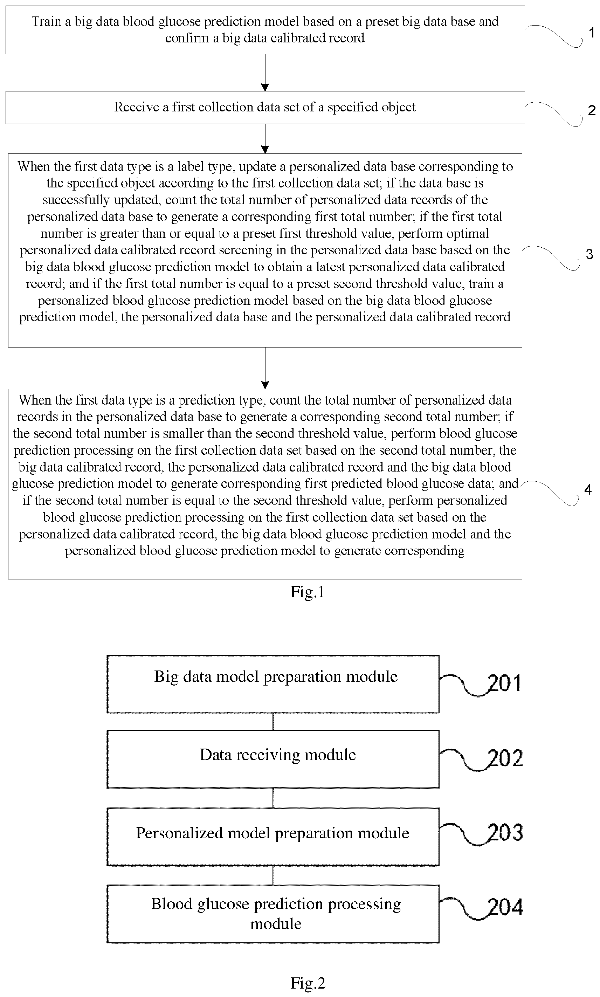

According to a blood glucose prediction method combining a big data model and a personalized model provided by Embodiment 1 of the present invention, a big data calibrated record and a training set are selected from a big data base first to train a big data blood glucose prediction model; after the big data blood glucose prediction model is well trained, collection data for received specified object are identified; if the received collection data are of a label type, the collection data are supplemented to a corresponding personalized data base as personalized data records, a personalized data calibrated record is screened out from the personalized data base when the number of records reaches a certain number (first threshold value), and when the number of records reaches a specified threshold value (second threshold value), the personalized data calibrated record is taken as a calibrated record of the big data blood glucose prediction model and the personalized data base is used as a training set to train a personalized blood glucose prediction model in combination with prediction results of the big data blood glucose prediction model; and if the received collection data are of a prediction type, blood glucose prediction is performed on the collection data by using the big data blood glucose prediction model calibrated with the big data calibrated record when the number of records < the first threshold value, by using the big data blood glucose prediction model calibrated with the personalized data calibrated record when the first threshold value ≤ the number of records < the second threshold value, and by using the big data blood glucose prediction model calibrated with the personalized data calibrated record and the personalized blood glucose prediction model when the number of records = the second threshold value. This enables the continual improvement of personalized prediction accuracy for specified objects by balancing big data prediction and the continuous updating of labeled data for these objects, thus elevating the level of prediction customization. Fig. 1 is a schematic diagram of a blood glucose prediction method combining a big data model and a personalized model according to Embodiment 1 of the invention. As shown in Fig. 1, the method mainly comprises the following steps.

In step 1, a big data blood glucose prediction model is trained based on a preset big data base and a big data calibrated record is confirmed.

Here, a neural network of the big data blood glucose prediction model adopts a fully connected network structure; the big data base comprises a plurality of big data records; the big data records comprise first preprandial sub-records and first postprandial sub-records; the first preprandial sub-record comprises a first collection time, a first time interval, a first optical data group, a first metabolic heat data group, a first physiological information data group and a first measured blood glucose level; the first postprandial sub-record comprises a second collection time, a second time interval, a second optical data group, a second metabolic heat data group, a second physiological information data group and a second measured blood glucose level; and the first and second measured blood glucose levels correspond to preprandial and postprandial measured blood glucose levels respectively.

Here, input data vectors of the big data blood glucose prediction model consist of unverified feature vectors, a big data preprandial calibration vector, a big data postprandial calibration vector, a big data preprandial calibrated blood glucose level and a big data postprandial calibrated blood glucose level. A neural network used in the model adopts a fully connected network structure. The model takes the big data preprandial calibration vector and the big data postprandial calibration vector as calibration reference vectors, and the big data preprandial calibrated blood glucose level and the big data postprandial calibrated blood glucose level as calibration reference blood glucose levels. A predicted value is obtained by performing fully connected computation on the unverified feature vectors through the fully connected network, and the predicted value is used as a floating value to obtain a final predicted blood glucose level based on the big data postprandial calibrated blood glucose level and the floating value.

The big data blood glucose prediction model needs to be trained based on a preset big data base before being used, the big data base stores a large number of big data records collected by different objects, and each big data record comprises a pair of preprandial and postprandial sub-records, that is, the first preprandial sub-record and the first postprandial sub-record, which correspond to collection information of an object before and after a meal. The first and second collection times are actual collection time information of a current sub-record. The first and second time intervals represent the time lapse between the current collection time and the previous mealtime for the object. The first and second optical data groups each comprise four optical signals, namely, PPG signals in three bands (1050 nm band, 940 nm band and 650 nm band) and an ambient light signal. The first and second metabolic heat data groups each comprise seven metabolic heat signals, namely, contact heat signal, human proximal temperature signal, human proximal humidity signal, human proximal radiant heat signal, radiant heat sensor calibration signal, human distal temperature signal and human distal humidity signal. The first and second physiological information data groups each comprise five types of physiological information, namely, age information, gender information, height information, weight information and body mass index (BMI) information. The first and second measured blood glucose levels correspond to actual measured blood glucose levels of the object before and after meals respectively. There are many ways to measure blood glucose levels, one of which is the conventional invasive measurement method, which can ensure the accuracy of measured values.

Step 1 comprises the following steps.

In step 11, the plurality of big data records are extracted from the big data base to form a big data set.

In step 12, first feature vector conversion processing is performed on each of the first preprandial sub-records or the first postprandial sub-records in the big data set to generate first feature vectors corresponding to each sub-record, and the first measured blood glucose levels or the second measured blood glucose levels corresponding to each of the first feature vectors are recorded as corresponding first feature blood glucose levels.

Here, the first feature vector conversion processing comprises: performing basic feature extraction processing on the first preprandial sub-records or the first postprandial sub-records to obtain corresponding time interval feature data, optical feature sequences, metabolic heat feature sequences and physiological feature sequences; identifying a preset first feature vector structure parameter; when the first feature vector structure parameter is a first structure, forming corresponding first feature vector outputs by the time interval feature data, the optical feature sequences and the metabolic heat feature sequences; and when the first feature vector structure parameter is a second structure, forming corresponding first feature vector outputs by the time interval feature data, the optical feature sequences, the metabolic heat feature sequences and the physiological feature sequences. Here, the first feature vector structure parameter defaults to the first structure.

The actual process involves converting collection data of each first preprandial sub-record or first postprandial sub-record in the big data set into first feature vectors matching an unverified feature vector structure required by the big data blood glucose prediction model, and first feature blood glucose levels corresponding to each first feature vector will be used for error calculation in the subsequent training step.

In step 13, a big data record is selected from the big data set as the big data calibrated record, two first feature vectors of the big data calibrated record are taken as a corresponding big data preprandial calibration vector and a big data postprandial calibration vector respectively, and two first feature blood glucose levels are taken as a corresponding big data preprandial calibrated blood glucose level and a big data postprandial calibrated blood glucose level respectively.

Here, it can be seen from the foregoing that the big data blood glucose prediction model has four calibration data: big data preprandial calibration vector, big data postprandial calibration vector, big data preprandial calibrated blood glucose level and big data postprandial calibrated blood glucose level. The current step is actually to select the big data calibrated record from the big data base to confirm the specific content of this group of calibration data.

It should be noted that there are many ways to select a big data record from the big data set as the big data calibrated record. One way is to screen all big data records in the big data set based on a group of preset normal preprandial blood glucose level ranges and normal postprandial blood glucose level ranges, to obtain a group of big data records featuring first and second measured blood glucose levels matching the preset normal preprandial blood glucose level ranges and normal postprandial blood glucose level ranges, from which any big data record is selected as the big data calibrated record.

In step 14, all the other first feature vectors except the big data preprandial calibration vector and the big data postprandial calibration vector are recorded as second feature vectors, and corresponding first model training vectors are formed by each of the second feature vectors, the big data preprandial calibration vector, the big data postprandial calibration vector, the big data preprandial calibrated blood glucose level and the big data postprandial calibrated blood glucose level.

Here, recording all the other first feature vectors except the big data preprandial calibration vector and the big data postprandial calibration vector as second feature vectors is to exclude the two first feature vectors corresponding to the big data calibrated record, and the second feature vectors are actually the unverified feature vectors in the input data vectors of the big data blood glucose prediction model mentioned above. As mentioned earlier, the input data vectors of the big data blood glucose prediction model consist of unverified feature vectors, a big data preprandial calibration vector, a big data postprandial calibration vector, a big data preprandial calibrated blood glucose level and a big data postprandial calibrated blood glucose level, so each first model training vector should consist of the corresponding second feature vector, the big data preprandial calibration vector, the big data postprandial calibration vector, the big data preprandial calibrated blood glucose level and the big data postprandial calibrated blood glucose level.

In step 15, based on the big data blood glucose prediction model, big data model prediction processing is performed on any of the first model training vectors to obtain corresponding first predicted data, and corresponding current predicted blood glucose data are generated according to the sum of the big data postprandial calibrated blood glucose level and the first predicted data; a training error of the current predicted blood glucose data in comparison with the first feature blood glucose level corresponding to the current first model training vector is calculated using a loss function of the big data blood glucose prediction model; when the training error does not meet a preset reasonable error range, the big data blood glucose prediction model is modulated based on the training error, and the next first model training vector is selected to continue training the modulated big data blood glucose prediction model; and training is stopped when the training error meets the reasonable error range.

Here, the big data blood glucose prediction model takes the big data preprandial calibration vector and the big data postprandial calibration vector as calibration reference vectors, and the big data preprandial calibrated blood glucose level and the big data postprandial calibrated blood glucose level as calibration reference blood glucose levels. The corresponding first predicted data are obtained by performing fully connected computation on the second feature vectors through the fully connected network, and the first predicted data are used as a floating value to obtain the current predicted blood glucose data based on the big data postprandial calibrated blood glucose level and the first predicted data. It is also known that each second feature vector corresponds to an actual measured blood glucose level, that is, the first feature blood glucose level. Then, by taking the current predicted blood glucose data as predicted data and the first feature blood glucose level as observation data, the training error between the corresponding current predicted blood glucose data and the corresponding first feature blood glucose level can be calculated based on the loss function of the big data blood glucose prediction model. There are many ways to realize the loss function here, such as mean square error loss function, exponential loss function, and cross entropy loss function, which will not be further elaborated here. After being obtained, the training error is evaluated against the preset reasonable error range; if it is within the reasonable error range, it means that the model has converged and training can be stopped; and if it is not within the reasonable error range, it means that the model has not converged, so it is necessary to modulate the big data blood glucose prediction model, and select the next first model training vector to continue training the modulated big data blood glucose prediction model. There are many ways to modulate the fully connected network of the big data blood glucose prediction model, such as using the back propagation algorithm based on gradient descent, which will not be further elaborated here.

At this point, the training of the big data blood glucose prediction model is completed by step 1, and then data can be collected for the individual collection object that needs long-term continuous blood glucose observation, that is, the specified object according to the data collection method in the following step 2.

In step 2, a first collection data set of a specified object is received.

Here, the first collection data set comprises a first data type, and the first data type is a prediction type or a label type; when the first data type is a label type, the first collection data set further comprises a first preprandial collection record and a first postprandial collection record; the first preprandial collection record comprises a third collection time, a third time interval, a third optical data group, a third metabolic heat data group, a third physiological information data group and a third measured blood glucose level; the first postprandial collection record comprises a fourth collection time, a fourth time interval, a fourth optical data group, a fourth metabolic heat data group, a fourth physiological information data group and a fourth measured blood glucose level; the third and fourth measured blood glucose levels correspond to preprandial and postprandial measured blood glucose levels respectively; and when the first data type is a prediction type, the first collection data set further comprises a fifth collection time, a fifth time interval, a fifth optical data group, a fifth metabolic heat data group and a fifth physiological information data group.

Here, the specified object is a designated individual object, and the first collection data set is a collection data set obtained by collecting optical signals, metabolic heat signals, physiological information and blood glucose information from the specified object.

There are two ways to collect data from the specified object: label data collection and prediction data collection. In case that the specified object selects the label data collection mode, a group of optical signals, metabolic heat signals, physiological information and measured blood glucose information before and after a meal of the specified object per time are collected to obtain a first collection data set consisting of a pair of first preprandial collection record and first postprandial collection record. In case that the specified object selects, the optical signals, metabolic heat signals and physiological information of the specified object at the current moment are collected to obtain a first collection data set.

In order to have a clearer understanding of the data structure of the received collection data set, the collection steps on the specified object side are described below.

In step A1, when the data collection mode is designated as the label data collection mode, a first data type is set as a label type; preprandial data collection and postprandial data collection are performed on the specified object to obtain a corresponding first preprandial collection record and a first postprandial collection record; and a corresponding first collection data set is formed by the obtained first data type, the first preprandial collection record and the first postprandial collection record.

Here, when performing preprandial data collection or postprandial data collection, the optical signals, metabolic heat signals and physiological information of the specified object are collected to obtain the corresponding collection time, time interval, optical data group, metabolic heat data group and physiological information data group, the current blood glucose information of the specified object is measured as the corresponding measured blood glucose level, and the obtained collection time, time interval, optical data group, metabolic heat data group, physiological information data group and measured blood glucose level form the corresponding first preprandial collection record or first postprandial collection record.

That is, the collection time, time interval, optical data group, metabolic heat data group, physiological information data group and measured blood glucose level obtained from the current preprandial data collection are taken as the third collection time, the third time interval, the third optical data group, the third metabolic heat data group, the third physiological information data group and the third measured blood glucose level of the first preprandial collection record; and the collection time, time interval, optical data group, metabolic heat data group, physiological information data group and measured blood glucose level obtained from the current postprandial data collection are taken as the fourth collection time, the fourth time interval, the fourth optical data group, the fourth metabolic heat data group, the fourth physiological information data group and the fourth measured blood glucose level of the first postprandial collection record. Then the first collection data set is formed by the first data type which is a label type, the first preprandial collection record and the first postprandial collection record.

In step A2, when the data collection mode is designated as the prediction data collection mode, the first data type is set as a prediction type. The optical signals, metabolic heat signals and physiological information of the specified object are collected to obtain the corresponding collection time, time interval, optical data group, metabolic heat data group and physiological information data group. The corresponding first collection data set is formed by the first data type and the obtained collection time, time interval, optical data group, metabolic heat data group and physiological information data group.

That is, the obtained collection time, time interval, optical data group, metabolic heat data group and physiological information data group are taken as the corresponding fifth collection time, the fifth time interval, the fifth optical data group, the fifth metabolic heat data group and the fifth physiological information data group, and the corresponding first collection data set is formed by the first data type which is a prediction type, the fifth collection time, the fifth time interval, the fifth optical data group, the fifth metabolic heat data group and the fifth physiological information data group.

It should be noted that in the above steps A1-A2, the processes of collecting the optical signals, metabolic heat signals and physiological information of the specified object to obtain the corresponding collection time, time interval, optical data group, metabolic heat data group and physiological information data group are similar, comprising the following steps.

In step B1, the current time is recorded as the corresponding collection time.

In step B2, the time lapse from the last meal time to the current time for the specified object is calculated as the corresponding time interval.

In step B3, one side of a PPG collection site of the specified object is illuminated by infrared light sources with three bands, and light intensity signals of the infrared light with three bands are collected on the other side to obtain corresponding PPG signals of the three bands; a light signal of the environment where the specified object is located is collected to obtain a corresponding ambient light signal; and the obtained PPG signals of the three bands and the ambient light signal form the corresponding optical data group.

Here, the PPG collection site is a preset collection site, often designated as the fingertip. If the PPG collection site is the fingertip, using infrared light sources with three bands to illuminate one side of the PPG collection site of the specified object is to illuminate one side of the fingertip of the specified object, and the top position of the finger (such as the nail area) is generally selected for illumination; and collecting the light intensity signals of the infrared light with three bands on the other side to obtain the corresponding PPG signals of the three bands is to collect signals on the other side of the fingertip of the specified object, and the finger pulp area (such as the finger pulp area vertically corresponding to the nail area) is generally selected for signal collection. Here, the infrared light with three bands is generally infrared light with a 1050 nm band, a 940 nm band and a 650 nm band, and the corresponding PPG signals of three bands are 1050 nm band PPG signal, 940 nm band PPG signal and 650 nm band PPG signal.

In step B4, a contact heat signal is collected at a contact heat collection site of the specified object to obtain the corresponding contact heat signal; human proximal temperature, humidity and radiant heat signals of the specified object are collected at a position that is no more than a preset first distance threshold value from the contact heat collection site to obtain the corresponding human proximal temperature signal, the human proximal humidity signal and the human proximal radiant heat signal; a calibration signal of a radiant heat sensor used for collecting radiant heat is collected to obtain the corresponding radiant heat sensor calibration signal; human distal temperature and humidity signals of the specified object are collected at a position that is more than a preset second distance threshold value from the contact heat collection site to obtain the corresponding human distal temperature signal and the human distal humidity signal; and the obtained contact heat signal, human proximal temperature signal, human proximal humidity signal, human proximal radiant heat signal, radiant heat sensor calibration signal, human distal temperature signal and human distal humidity signal form the corresponding metabolic heat data group.

Here, the first distance threshold value is smaller than the second distance threshold value; the first distance threshold value is a proximal threshold value, and a position that does not exceed the first distance threshold value is regarded as a proximal position; and the second distance threshold value is a distal threshold value, and a position that exceeds the second distance threshold value is regarded as a distal position.

Here, the contact heat collection site is related to a PPG signal collection position. If the PPG signal collection position is the finger pulp area of the fingertip, then the contact heat collection site is the pulp epidermis position on the fingertip, the corresponding contact heat signal or human proximal radiant heat signal are two heat energy signals collected at the distance which is 0 from the pulp epidermis position or is greater than 0 and does not exceed the first distance threshold value, the corresponding human proximal temperature signal or human distal temperature signal are two temperature change signals collected at the distance which is greater than 0 from the pulp epidermis position and does not exceed the first distance threshold value or the distance which exceeds the second distance threshold value, the corresponding human proximal humidity signal or human distal humidity signal are two humidity change signals collected at the distance which is greater than 0 from the pulp epidermis position and does not exceed the first distance threshold value or the distance which exceeds the second distance threshold value, and the radiant heat sensor calibration signal is an ambient heat energy signal collected for calibrating heat energy signals of the human body at the distance from the pulp epidermis position exceeding the second distance threshold value.

In step B5, the age information, gender information, height information, weight information and BMI information of the specified object are collected to form the corresponding physiological information data group.

To sum up, through the above steps B1-B5, the optical signals, metabolic heat signals, physiological information and blood glucose information of the specified object can be collected according to the specified data collection mode to generate the corresponding first collection data set.

In step 3, when the first data type is a label type, a personalized data base corresponding to the specified object is updated according to the first collection data set; if the data base is successfully updated, the total number of personalized data records of the personalized data base is counted to generate a corresponding first total number; if the first total number is greater than or equal to a preset first threshold value, optimal personalized data calibrated record screening is performed in the personalized data base based on the big data blood glucose prediction model to obtain a latest personalized data calibrated record; and if the first total number is equal to a preset second threshold value, a personalized blood glucose prediction model is trained based on the big data blood glucose prediction model, the personalized data base and the personalized data calibrated record.

Here, the first threshold value is smaller than the second threshold value, and a model structure of the personalized blood glucose prediction model is a support vector machine model structure, a random forest model structure or a decision tree model structure. The personalized data base comprises the plurality of personalized data records; the personalized data records comprise second preprandial sub-records and second postprandial sub-records; the second preprandial sub-record comprises a sixth collection time, a sixth time interval, a sixth optical data group, a sixth metabolic heat data group, a sixth physiological information data group and a fifth measured blood glucose level; the second postprandial sub-record comprises a seventh collection time, a seventh time interval, a seventh optical data group, a seventh metabolic heat data group, a seventh physiological information data group and a sixth measured blood glucose level; the fifth and sixth measured blood glucose levels correspond to preprandial and postprandial measured blood glucose levels respectively; and the maximum total number of records in the personalized data base is the second threshold value.

Here, when the first data type is the label type, it means that the first collection data set currently obtained is label data, and the personalized data base needs to be updated with the collected data; the total number of personalized data records in the updated personalized data base is counted; if the total number is smaller than the first threshold value, it means that the total volume of the label data is insufficient for screening the personalized data calibrated records; if the total number exceeds the first threshold value, it means that the total volume of the label data is enough to screen the personalized data calibrated records and then select a personalized data calibrated record; and if the total number reaches the second threshold value, it means that the total volume of the label data in the personalized data base is full and the personalized blood glucose prediction model can be trained. The personalized blood glucose prediction model in Embodiment of the present invention is used in combination with the big data blood glucose prediction model, so when training the personalized blood glucose prediction model, the calibration data of the big data blood glucose prediction model need to be confirmed by using the personalized data calibrated record first, then other records than the personalized data calibrated record are selected from the personalized data base as training data, and then the personalized blood glucose prediction model is trained in combination with the prediction results of the big data blood glucose prediction model. The model structure of the personalized blood glucose prediction model adopts a support vector machine model structure, a random forest model structure or a decision tree model structure. Compared with the fully connected network structure, these three structures have the advantage of being able to refine input vectors based on individual characteristics through creating conditional branches to achieve personalized output. The personalized blood glucose prediction model is to further refine the prediction results of the big data blood glucose prediction model, and its input data structure comprises: part of the features of a big data model unverified feature vector (new unverified feature vector), part of the features of the big data preprandial calibration vector (personalized preprandial calibration vector), part of the features of the big data postprandial calibration vector (personalized postprandial calibration vector), the big data preprandial calibrated blood glucose level, the big data postprandial calibrated blood glucose level, and the predicted blood glucose data of the big data model. Its output result is also a floating value, and the final blood glucose predicted data can be obtained based on the floating value and the predicted blood glucose data of the big data model.

Step 3 comprises the following steps.

In step 31, a personalized data base corresponding to the specified object is updated according to the first collection data set, comprising the following steps.

In step 311, the total number of personalized data records in the personalized data base is counted to generate a corresponding current total number; when the current total number is smaller than the second threshold value, a new personalized data record is added as a current record in the personalized data base; and when the current total number is equal to the second threshold value, the personalized data record which is the earliest added in the personalized data base is taken as the current record.

In step 312, the first preprandial collection record is extracted from the first collection data set as the second preprandial sub-record of the current record and stored in the personalized data base, and the first postprandial collection record is extracted as the second postprandial sub-record of the current record and stored in the personalized data base.

Here, the maximum total number of records in the personalized data base is the second threshold value, and if the second threshold value is 10 , it means that the personalized data base can only store 10 personalized data records at most. When updating the personalized data base corresponding to the specified object, if the total number of the personalized data records in the data base is not full, that is, the current total number is smaller than the second threshold value, Embodiment 1 of the present invention provides that a new personalized data record will be added to the personalized data base based on the first collection data set; and if the total number of the personalized data records in the data base is full, that is, the current total number is equal to the second threshold value,Embodiment 1 of the present invention provides that the personalized data record with the earliest addition time in the personalized data base is updated based on the first collection data set.

In step 32, if the data base is successfully updated, the total number of personalized data records of the personalized data base is counted to generate a corresponding first total number.

Here, according to Embodiment 1 of the present invention, following each successful update of the data base, the latest total number of records in the personalized data base, that is, the first total number, is identified; if the first total number is greater than or equal to the first threshold value, it means that the total volume of the label data is enough to screen the personalized data calibrated records, and then the latest personalized data calibrated record is selected through the subsequent step 33, so that the personalized data calibrated record can be continuously updated; and if the first total number reaches the second threshold value, it means that the total volume of the label data in the personalized data base is full, and model training can be performed through the subsequent step 34.

In step 33, if the first total number is greater than or equal to a preset first threshold value, the step that screening an optimal personalized data calibrated record is performed in the personalized data base based on the big data blood glucose prediction model to obtain a latest personalized data calibrated record comprises the following steps.

In step 331, first feature vector conversion processing is performed on the second preprandial sub-record or the second postprandial sub-record of each of the personalized data records in the personalized data base to generate third feature vectors corresponding to each sub-record, and the fifth measured blood glucose levels or the sixth measured blood glucose levels corresponding to each of the third feature vectors are recorded as corresponding second feature blood glucose levels.

Here, similar to the aforementioned step 12, the first feature vector conversion processing comprises: performing basic feature extraction processing on the second preprandial sub-records or the second postprandial sub-records to obtain corresponding time interval feature data, optical feature sequences, metabolic heat feature sequences and physiological feature sequences; identifying a first feature vector structure parameter; when the first feature vector structure parameter is a first structure, forming corresponding third feature vector outputs by the time interval feature data, the optical feature sequences and the metabolic heat feature sequences; and when the first feature vector structure parameter is a second structure, forming corresponding third feature vector outputs by the time interval feature data, the optical feature sequences, the metabolic heat feature sequences and the physiological feature sequences. Here, the first feature vector structure parameter defaults to the first structure.

In step 332, the personalized data records are sequentially extracted as a current calibrated record in the personalized data base; two third feature vectors of the current calibrated record are taken as a corresponding big data preprandial calibration vector and a big data postprandial calibration vector respectively, and two second feature blood glucose levels are taken as a corresponding big data preprandial calibrated blood glucose level and a big data postprandial calibrated blood glucose level respectively; third feature vectors of other personalized data records except the current calibrated record are recorded as fourth feature vectors, and corresponding first model input vectors are formed by each of the fourth feature vectors, the big data preprandial calibration vector, the big data postprandial calibration vector, the big data preprandial calibrated blood glucose level and the big data postprandial calibrated blood glucose level; based on the big data blood glucose prediction model, big data model prediction processing is performed on each of the first model input vectors to obtain corresponding second predicted data, and corresponding first model predicted blood glucose data are generated according to the sum of the big data postprandial calibrated blood glucose level and the second predicted data; an absolute difference between each of the first model predicted blood glucose data and the second feature blood glucose level corresponding to a current first model input vector is calculated to obtain a corresponding first prediction error; and the sum of all the obtained first prediction errors is calculated to obtain a first prediction error sum corresponding to the current calibrated record.

For example, the first threshold value is 4, the personalized data base has 4 personalized data records, that is, personalized data records 1-4, and each personalized data record corresponds to 2 third feature vectors, so 8 third feature vectors 1-8 are obtained; each third feature vector corresponds to 1 second feature blood glucose level, so 8 second feature blood glucose levels 1-8 are obtained;
when the personalized data record 1 is taken as the current calibrated record, the corresponding third feature vectors 1-2 and the second feature blood glucose levels 1-2 are taken as the four calibration data of the big data blood glucose prediction model; the third feature vectors 3-8 are taken as the corresponding fourth feature vectors 1-6, and measured blood glucose levels corresponding to the fourth feature vectors 1-6 are the second feature blood glucose levels 3-8; 6 first model input vectors 1-6 can be obtained by the fourth feature vectors and the four calibration data; the first model input vectors 1-6 are sequentially sent to the big data blood glucose prediction model for big data model prediction processing to obtain 6 corresponding second predicted data 1-6, and 6 first model predicted blood glucose data 1-6 can be obtained based on the calculation relationship of "second predicted data + big data postprandial calibrated blood glucose level = first model predicted blood glucose data"; after obtaining the first model predicted blood glucose data 1-6, 6 first prediction errors 1-6 can be obtained by taking the absolute value of (first model predicted blood glucose data i - second feature blood glucose level (i+2)), where 1 ≤ i ≤ 6; the sum of the first prediction errors 1-6 is calculated to obtain a first prediction error sum 1 corresponding to the personalized data record 1; and
similarly, when the personalized data records 2-4 are taken as the current calibrated record, corresponding first prediction error sums 2-4 are obtained.

In step 333, a minimum prediction error sum is taken as a principle for screening an optimal calibration personalized record group , and the personalized data record with the minimum first prediction error sum in the personalized data base is taken as the latest personalized data calibrated record.

For example, among the first prediction error sums 1-4 corresponding to the personalized data records 1-4, the first prediction error sum 1 is the minimum value, so the minimum prediction error sum is taken as the principle for screening the optimal calibration personalized record group , the personalized data calibrated record finally selected from the personalized data base should be the personalized data record 1.

In step 34, if the first total number is equal to a preset second threshold value, the step for training a personalized blood glucose prediction model based on the big data blood glucose prediction model, the personalized data base and the personalized data calibrated record comprises the following steps.

In step 341, first feature vector conversion processing is performed on the second preprandial sub-record or the second postprandial sub-record of each of the personalized data records in the personalized data base to generate fifth feature vectors corresponding to each sub-record, and the fifth measured blood glucose levels or the sixth measured blood glucose levels corresponding to each of the fifth feature vectors are recorded as corresponding third feature blood glucose levels.

Here, the first feature vector conversion processing is similar to that in step 331, and it will not be further described here.

In step 342, two fifth feature vectors of the personalized data calibrated record are taken as a corresponding big data preprandial calibration vector and a big data postprandial calibration vector respectively, and two third feature blood glucose levels are taken as a corresponding big data preprandial calibrated blood glucose level and a big data postprandial calibrated blood glucose level respectively.

Here, because the personalized data calibrated record has been selected by the aforementioned step 33, 2 fifth feature vectors and 2 third feature blood glucose levels of the personalized data calibrated record are used as four calibration data of the big data blood glucose prediction model.

In step 343, the fifth feature vectors of other personalized data records except the personalized data calibrated record in the personalized data base are recorded as sixth feature vectors, and corresponding second model training vectors are formed by each of the sixth feature vectors, the big data preprandial calibration vector, the big data postprandial calibration vector, the big data preprandial calibrated blood glucose level and the big data postprandial calibrated blood glucose level.

In step 344, big data model prediction processing is performed on each of the second model training vectors based on the big data blood glucose prediction model to obtain corresponding third predicted data, and corresponding big data predicted blood glucose data are generated according to the sum of the big data postprandial calibrated blood glucose level and the third predicted data.

For example, if the second threshold value is 10, the personalized data base has 10 personalized data records, that is, personalized data records 1-10, and each personalized data record corresponds to 2 fifth feature vectors, so 20 fifth feature vectors 1-20 are obtained; each fifth feature vector corresponds to 1 third feature blood glucose level, so 20 third feature blood glucose levels 1-20 are obtained; because the personalized data calibrated record is known as the personalized data record 1, the four calibration data of the big data blood glucose prediction model are the fifth feature vectors 1-2 and the third feature blood glucose levels 1-2; the fifth feature vectors 3-20 are taken as the corresponding sixth feature vectors 1-18, and the measured blood glucose levels corresponding to the sixth feature vectors 1-18 are the third feature blood glucose levels 3-20; 18 second model training vectors 1-18 can be obtained by the sixth feature vectors and the four calibration data; and based on the big data blood glucose prediction model, the second model training vectors 1-18 can be subjected to big data model prediction processing to obtain 18 third predicted data 1-18, and 18 big data predicted blood glucose data 1-18 can be obtained based on the calculation relationship of "big data postprandial calibrated blood glucose value + third predicted data = big data predicted blood glucose data".

In step 345, according to a preset big data-personalized feature association status, part of feature data are extracted from the sixth feature vectors corresponding to each of the second model training vectors to form corresponding seventh feature vectors, part of feature data are extracted from the big data preprandial calibration vector to form a corresponding personalized preprandial calibration vector, and part of feature data are extracted from the big data postprandial calibration vector to form a corresponding personalized postprandial calibration vector; and corresponding third model training vectors are formed by the seventh feature vectors, the personalized preprandial calibration vector, the personalized postprandial calibration vector, the big data preprandial calibrated blood glucose level, the big data postprandial calibrated blood glucose level and the big data predicted blood glucose data.

Here, the big data-personalized feature association status comprises first, second, third and fourth status bits, the matching feature data of the first status bit are time interval feature data, the matching feature data of the second status bit are optical feature sequences, the matching feature data of the third status bit are metabolic heat feature sequences, and the matching feature data of the fourth status bit are physiological feature sequences.

The step for, according to a preset big data-personalized feature association status, extracting part of feature data from the sixth feature vectors corresponding to each of the second model training vectors to form corresponding seventh feature vectors, extracting part of feature data from the big data preprandial calibration vector to form a corresponding personalized preprandial calibration vector, and extracting part of feature data from the big data postprandial calibration vector to form a corresponding personalized postprandial calibration vector, comprises: identifying whether each status bit of the big data-personalized feature association status is an association status, and if so, adding matching feature data of each status bit in the sixth feature vectors, the big data preprandial calibration vector and the big data postprandial calibration vector to the corresponding seventh feature vectors, the personalized preprandial calibration vector and the personalized postprandial calibration vector.

Here, the personalized blood glucose prediction model is to further refine the prediction results of the big data blood glucose prediction model, and its input data structure, that is, the data structure of the third model training vectors, comprises: part of the features of big data model unverified feature vectors (that is, the seventh feature vectors)+ part of the features of the big data preprandial calibration vector (personalized preprandial calibration vector)+ part of the features of the big data postprandial calibration vector (personalized postprandial calibration vector)+the big data preprandial calibrated blood glucose level+ the big data postprandial calibrated blood glucose level + the big data predicted blood glucose data. Here, the seventh feature vectors, the personalized preprandial calibration vector and the personalized postprandial calibration vector are feature vectors reconstructed from part of the features extracted from the corresponding big data model unverified feature vectors, that is, the sixth feature vectors, the big data preprandial calibration vector and the big data postprandial calibration vector. The way to extract part of the features here is related to the four status bits of the preset big data-personalized feature association status.

For example, if the first, second, third and fourth status bits of the big data-personalized feature association status are respectively association status, association status, association status and non-association status, then time interval feature data, optical feature sequences and metabolic heat feature sequences are extracted from the sixth feature vectors to form the seventh feature vectors, time interval feature data, optical feature sequences and metabolic heat feature sequences are extracted from the big data preprandial calibration vector to form the personalized preprandial calibration vector, and time interval feature data, optical feature sequences and metabolic heat feature sequences are extracted from the big data postprandial calibration vector to form the personalized postprandial calibration vector.

For another example, it is known that there are second model training vectors 1-18 and corresponding 18 big data predicted blood glucose data 1-18, and the sixth feature vectors 1-18, the big data preprandial calibration vector and the big data postprandial calibration vector in the second model training vectors 1-18 can be subjected to partial feature data extraction according to the big data-personalized feature association status, to obtain 18 new seventh feature vectors 1-18, a new personalized preprandial calibration vector and a new personalized postprandial calibration vector; and then, based on the fact that the the third model training vectors are formed by the seventh feature vectors, the personalized preprandial calibration vector, the personalized postprandial calibration vector, the big data preprandial calibrated blood glucose level, the big data postprandial calibrated blood glucose level, and the big data predicted blood glucose data, 18 third model training vectors 1-18 can be obtained.

In step 346, personalized model prediction processing is performed on any one of the third model training vectors based on the personalized blood glucose prediction model to obtain corresponding fourth predicted data, and corresponding current predicted blood glucose data are generated according to the sum of the big data predicted blood glucose data and the fourth predicted data; an absolute difference between the current predicted blood glucose data and the third feature blood glucose level corresponding to a current third model training vector is calculated as a current measurement error; the personalized blood glucose prediction model is modulated when the current measurement error does not match a preset reasonable error range; and then the next third model training vector is selected to train the personalized blood glucose prediction model until training with the last third model training vector is completed.

For example, it is known that there are 18 third model training vectors 1-18, corresponding 18 big data predicted blood glucose data 1-18 and 18 third feature blood glucose levels 3-20;
then, personalized model prediction processing is performed on the third model training vector 1 based on the personalized blood glucose prediction model to obtain fourth predicted data 1, the current predicted blood glucose data 1 are calculated as the sum of the big data predicted blood glucose data 1 and the fourth predicted data 1, an absolute difference between the current predicted blood glucose data 1 and the corresponding third feature blood glucose level 3 is calculated as a current measurement error 1, and the personalized blood glucose prediction model is modulated if the current measurement error 1 does not match the preset reasonable error range; then, personalized model prediction processing is performed on the third model training vector 2 based on the personalized blood glucose prediction model to obtain fourth predicted data 2, the current predicted blood glucose data 2 are calculated as the sum of the big data predicted blood glucose data 2 and the fourth predicted data 2, an absolute difference between the current predicted blood glucose data 2 and the corresponding third feature blood glucose level 4 is calculated as a current measurement error 2, and the personalized blood glucose prediction model is modulated if the current measurement error 2 does not match the preset reasonable error range; and so on, until training with the third model training vector 18 is completed, training of the personalized blood glucose prediction model is finished.

From the above step 3, it can be seen that in Embodiment 1 of the present invention, every time the personalized data base is updated (new records are added or old records are replaced), the current total number of records in the personalized data base will be identified, the personalized data calibrated record is automatically updated if the total number of records exceeds the first threshold value, and if the total number of records has reached the second threshold value, a training session for the personalized blood glucose prediction model is conducted. Through this processing method, personalized calibration of the big data blood glucose prediction model can be continuously performed by using the optimal personalized data calibrated record, and the personalized blood glucose prediction model can be continuously trained by using a plurality of latest label data of a specified user to further match the latest state of the specified user.

In step 4, when the first data type is a prediction type, the total number of personalized data records in the personalized data base is counted to generate a corresponding second total number; if the second total number is smaller than the second threshold value, blood glucose prediction processing is performed on the first collection data set based on the second total number, the big data calibrated record, the personalized data calibrated record and the big data blood glucose prediction model to generate corresponding first predicted blood glucose data; and if the second total number is equal to the second threshold value, personalized blood glucose prediction processing is performed on the first collection data set based on the personalized data calibrated record, the big data blood glucose prediction model and the personalized blood glucose prediction model to generate corresponding second predicted blood glucose data.

Here, when the first data type is a prediction type, it means that the first collection data set currently collected is the data to be predicted, and is obtained by collecting the optical signal, metabolic heat signal and physiological information of the specified object at the current moment; in this case, according to Embodiment 1 of the present invention, the total number of personalized data records in the personalized data base is counted; if the total number is smaller than the second threshold value, it means that the personalized blood glucose prediction model has not been trained, so only the big data blood glucose prediction model calibrated with the big data calibrated record or the personalized data calibrated record can be used for prediction; and if the total number is equal to the second threshold value, it means that the personalized blood glucose prediction model has been trained at least once, so the big data blood glucose prediction model and the personalized blood glucose prediction model calibrated with the personalized data calibrated record are used for prediction instead.

Step 4 comprises the following steps.

In step 41, the total number of personalized data records of the personalized data base is counted to generate a corresponding second total number.

In step 42, if the second total number is smaller than the second threshold value, the step forfperforming blood glucose prediction processing on the first collection data set based on the second total number, the big data calibrated record, the personalized data calibrated record and the big data blood glucose prediction model to generate corresponding first predicted blood glucose data comprises the following steps.

In step 421, whether the second total number is smaller than the first threshold value is determined; if the second total number is smaller than the first threshold value, first feature vector conversion processing is performed on the first preprandial sub-record and the first postprandial sub-record of the big data calibrated record, two obtained feature vectors are taken as a corresponding big data preprandial calibration vector and a big data postprandial calibration vector, and the first measured blood glucose level and the second measured blood glucose level of the big data calibrated record are taken as a corresponding big data preprandial calibrated blood glucose level and a big data postprandial calibrated blood glucose level; and if the second total number is greater than or equal to the first threshold value, first feature vector conversion processing is performed on the second preprandial sub-record and the second postprandial sub-record of the personalized data calibrated record, two obtained feature vectors are taken as a corresponding big data preprandial calibration vector and a big data postprandial calibration vector, and the fifth measured blood glucose level and the sixth measured blood glucose level of the personalized data calibrated record are taken as the corresponding big data preprandial calibrated blood glucose level and the big data postprandial calibrated blood glucose level.

Here, referring to the aforementioned step 3, we know that when the second total number is smaller than the first threshold value, the personalized blood glucose prediction model has not been well trained, and the personalized data calibrated record has not been screened out, so in this case, only the big data calibrated record can be used to set the 4 calibration data of the big data blood glucose prediction model; and when the second total number is greater than or equal to the first threshold value and smaller than the second threshold value, the personalized blood glucose prediction model has not been trained, but the personalized data calibrated record has been screened out, so in this case, the personalized data calibrated record is used to set the 4 calibration data of the big data blood glucose prediction model.

In step 422, first feature vector conversion processing is performed on the first collection data set to generate corresponding eighth feature vectors, and corresponding second model input vectors are formed by the eighth feature vectors, the big data preprandial calibration vector, the big data postprandial calibration vector, the big data preprandial calibrated blood glucose level and the big data postprandial calibrated blood glucose level.

Here, similar to the aforementioned step 12, the first feature vector conversion processing comprises: performing basic feature extraction processing on the first collection data set to obtain corresponding time interval feature data, optical feature sequences, metabolic heat feature sequences and physiological feature sequences; identifying a first feature vector structure parameter; when the first feature vector structure parameter is a first structure, forming corresponding eighth feature vector outputs by the time interval feature data, the optical feature sequences and the metabolic heat feature sequences; and when the first feature vector structure parameter is a second structure, forming corresponding eighth feature vector outputs by the time interval feature data, the optical feature sequences, the metabolic heat feature sequences and the physiological feature sequences. Here, the first feature vector structure parameter defaults to the first structure.

Further, as mentioned earlier, the structure of the input data vectors of the big data blood glucose prediction model should be unverified feature vectors + a big data preprandial calibration vector+ a big data postprandial calibration vector+a big data preprandial calibrated blood glucose level + a big data postprandial calibrated blood glucose level, so the structure of second model input vectors should be the eighth feature vectors+ the big data preprandial calibration vector+the big data postprandial calibration vector+ the big data preprandial calibrated blood glucose level + the big data postprandial calibrated blood glucose level.

In step 423, big data model prediction processing is performed on the second model input vectors based on the big data blood glucose prediction model to obtain corresponding fifth predicted data, and corresponding first predicted blood glucose data are generated according to the sum of the big data postprandial calibrated blood glucose level and the fifth predicted data.

Here, from the relationship between an output predicted value of the big data blood glucose prediction model and a final predicted value mentioned above: the final predicted value = the output predicted value + the big data postprandial calibrated blood glucose value, we can know that the first predicted blood glucose data = the fifth predicted data + the big data postprandial calibrated blood glucose value.

In step 43, if the second total number is equal to the second threshold value, the step for performing personalized blood glucose prediction processing on the first collection data set based on the personalized data calibrated record, the big data blood glucose prediction model and the personalized blood glucose prediction model to generate corresponding second predicted blood glucose data comprises the following steps.

In step 431, first feature vector conversion processing is performed on the second preprandial sub-record and the second postprandial sub-record of the personalized data calibrated record to obtain two ninth feature vectors, the two ninth feature vectors are taken as a corresponding big data preprandial calibration vector and a big data postprandial calibration vector respectively, and the fifth and sixth measured blood glucose levels of the personalized data calibrated record are taken as a corresponding big data preprandial calibrated blood glucose level and a big data postprandial calibrated blood glucose level respectively.

Here, the first feature vector conversion processing is similar to that in step 331, and it will not be further described here.

In addition, referring to the aforementioned step 3, we know that when the second total number is equal to the second threshold value, the personalized data calibrated record has been screened out and the personalized blood glucose prediction model has been trained at least once, so in this case, the personalized data calibrated record should be used to set the 4 calibration data of the big data blood glucose prediction model.

In step 432, first feature vector conversion processing is performed on the first collection data set to generate corresponding tenth feature vectors, and corresponding third model input vectors are formed by the tenth feature vectors, the big data preprandial calibration vector, the big data postprandial calibration vector, the big data preprandial calibrated blood glucose level and the big data postprandial calibrated blood glucose level.

Here, the first feature vector conversion processing is similar to the first feature vector conversion processing in step 422, and it will not be further described here.

Further, from the structure of the input data vectors of the big data blood glucose prediction model mentioned above, it can be known that the structure of the third model input vectors should be the tenth feature vectors+the big data preprandial calibration vector+the big data postprandial calibration vector+ the big data preprandial calibrated blood glucose level +the big data postprandial calibrated blood glucose level.

In step 433, big data model prediction processing is performed on the third model input vectors based on the big data blood glucose prediction model to obtain corresponding sixth predicted data, and corresponding big data predicted blood glucose data are generated according to the sum of the big data postprandial calibrated blood glucose level and the sixth predicted data.

Here, the third model input vectors are predicted by using the big data blood glucose prediction model calibrated with the personalized data calibrated record, and from the relationship between an output predicted value of the big data blood glucose prediction model and a final predicted value mentioned above: the final predicted value = the output predicted value + the big data postprandial calibrated blood glucose value, we can know that the big data predicted blood glucose data = the sixth predicted data + the big data postprandial calibrated blood glucose value.

In step 434, according to a preset big data-personalized feature association status, part of feature data are extracted from the tenth feature vectors to form corresponding eleventh feature vectors, part of feature data are extracted from the big data preprandial calibration vector to form a corresponding personalized preprandial calibration vector, and part of feature data are extracted from the big data postprandial calibration vector to form a corresponding personalized postprandial calibration vector.

Here, extraction of partial feature data is similar to the previous step 345, comprising: identifying whether each status bit of the big data-personalized feature association status is an association status, and if so, adding matching feature data of each status bit in the tenth feature vectors, the big data preprandial calibration vector and the big data postprandial calibration vector to the corresponding eleventh feature vectors, the personalized preprandial calibration vector and the personalized postprandial calibration vector.

In step 435, corresponding fourth model input vectors are formed by the eleventh feature vectors, the personalized preprandial calibration vector, the personalized postprandial calibration vector, the big data preprandial calibrated blood glucose level, the big data postprandial calibrated blood glucose level and the big data predicted blood glucose data.

Here, it can be known from the foregoing that the structure of input data of the personalized blood glucose prediction model, that is, the data structure of the fourth model input vectors comprises: part of the features of big data model unverified feature vectors (the eleventh feature vectors),+ part of the features of the big data preprandial calibration vector (personalized preprandial calibration vector), +part of the features of the big data postprandial calibration vector (personalized postprandial calibration vector), +the big data preprandial calibrated blood glucose level, +the big data postprandial calibrated blood glucose level, +the big data predicted blood glucose data.

In step 436, personalized model prediction processing is performed on the fourth model input vectors based on the personalized blood glucose prediction model to obtain corresponding seventh predicted data, and the corresponding second predicted blood glucose data are generated according to the sum of the big data predicted blood glucose data and the seventh predicted data.

Here, the second predicted blood glucose data = the big data predicted blood glucose data + the seventh predicted data = the big data postprandial calibrated blood glucose level + the sixth predicted data + the seventh predicted data. That is to say, when personalized blood glucose prediction is performed on the collection data set of the specified object by using the big data blood glucose prediction model and the personalized blood glucose prediction model, the two models each predict a floating difference, and the final personalized blood glucose predicted data can be obtained by adding the two floating differences on top of the big data postprandial calibrated blood glucose level calibrated with the personalized data calibrated record.

It should be noted that the first feature vector conversion processing mentioned in the previous steps is implemented in a similar way, comprising: taking the first preprandial sub-record, the first postprandial sub-record, the second preprandial sub-record, the second postprandial sub-record or the first collection data set for the first feature vector conversion processing all as current records; performing basic feature extraction processing on the current records to obtain corresponding time interval feature data, optical feature sequences, metabolic heat feature sequences and physiological feature sequences; identifying a preset first feature vector structure parameter; when the first feature vector structure parameter is a first structure, forming corresponding feature vector outputs by the time interval feature data, the optical feature sequences and the metabolic heat feature sequences; and when the first feature vector structure parameter is a second structure, forming corresponding feature vector outputs by the time interval feature data, the optical feature sequences, the metabolic heat feature sequences and the physiological feature sequences. Here, the first feature vector structure parameter defaults to the first structure. It should be noted that the step of performing basic feature extraction processing on the current records to obtain corresponding time interval feature data, optical feature sequences, metabolic heat feature sequences and physiological feature sequences comprises the following steps.

In step C1, a time interval of the current record is taken as the time interval feature data.

In step C2, an optical data group of the current record is taken as a first optical feature group; DC feature extraction is performed on PPG signals of three bands in the first optical feature group to obtain three corresponding PPG DC feature data, AC feature extraction is performed on the PPG signals of three bands to obtain three corresponding PPG AC feature data, average peak-to-peak distance calculation is performed on the PPG signals of three bands to obtain three corresponding PPG peak-to-peak distance feature data, and average valley-to-valley distance calculation is performed on the PPG signals of three bands to obtain three corresponding PPG valley-to-valley distance feature data; the mean value of a central segment signal of an ambient light signal in the first optical feature group is calculated to obtain a corresponding ambient light feature datum; an optical feature sequence with thirteen pieces of feature information is formed by the obtained three PPG DC feature data, three PPG AC feature data, three PPG peak-to-peak distance feature data, three PPG valley-to-valley distance feature data and one ambient light feature datum; and normalizing feature data of the optical feature sequence.

Here, the DC feature extraction comprises: performing low-pass filtering on the PPG signals of three bands according to a specified cutoff frequency to obtain three corresponding low-pass filtered signals, and performing average calculation on the three low-pass filtered signals to obtain three corresponding PPG DC feature data. The AC feature extraction comprises: intercepting sub-signals with specified lengths from the middle positions of the PPG signals of three bands as three corresponding sub-signals, performing band-pass filtering on the three sub-signals according to a specified passband to obtain three corresponding band-pass filtered signals, calculating a difference between a mean peak value and a mean valley value of each band-pass filtered signal as a corresponding first difference, and taking the obtained three first differences as three corresponding PPG AC feature data. When extracting the central segment signal of the ambient light signal, a sub-signal with a specified length is intercepted from the middle position of the ambient light signal as the central segment signal. For example, if the specified length is 20 seconds, then the central segment signal is a signal segment of 20 seconds in the middle of the ambient light signal. In addition, there are many ways to normalize the feature data of the optical feature sequence, such as Min-Max scaling and Z-score standardization, which will not be further describehere.

In step C3, the metabolic heat data group of the current record is taken as a first metabolic heat feature group; the mean value of a tail signal of a contact heat signal of the first metabolic heat feature group is calculated to obtain corresponding contact heat feature data; the mean value of a tail signal of a human proximal temperature signal of the first metabolic heat feature group is calculated to obtain corresponding proximal temperature feature data; the mean value of a tail signal of a human proximal humidity signal of the first metabolic heat feature group is calculated to obtain corresponding proximal humidity feature data; the mean value of a tail signal of a human proximal radiant heat signal of the first metabolic heat feature group is calculated to obtain corresponding proximal radiant heat feature data; the mean value of a tail signal of a radiant heat sensor calibration signal of the first metabolic heat feature group is calculated to obtain corresponding radiant heat calibration feature data; the mean value of a tail signal of a human distal temperature signal of the first metabolic heat feature group is calculated to obtain corresponding distal temperature feature data; the mean value of a tail signal of a human distal humidity signal of the first metabolic heat feature group is calculated to obtain corresponding distal humidity feature data; a metabolic heat feature sequence with seven pieces of feature information is formed by the obtained contact heat feature data, proximal temperature feature data, proximal humidity feature data, proximal radiant heat feature data, radiant heat calibration feature data, distal temperature feature data and distal humidity feature data; and the feature data of the metabolic heat feature sequence are normalized.

Here, the tail signal of each metabolic heat signal in the above steps refers to a segment of the current signal with a specified length at the end of the signal. If the specified length is 10 seconds, then the tail signal of each metabolic heat signal refers to the signal segment of the last 10 seconds of the current signal. In addition, there are many ways to normalize eachefeature data of the metabolic heat feature sequence, such as Min-Max scaling and Z-score standardization, similar to step B2, which will not be further described here.

In step C4, the physiological information data group of the current record is taken as a first physiological feature group; age information in the first physiological feature group is taken as corresponding age feature data; numerical conversion is performed on gender information in the first physiological feature group to obtain corresponding gender feature data; height information in the first physiological feature group is taken as corresponding height feature data; weight information in the first physiological feature group is taken as corresponding weight feature data; BMI information in the first physiological feature group is taken as corresponding BMI feature data; and a physiological feature sequence with five pieces of feature information is formed by the obtained age feature data, gender feature data, height feature data, weight feature data and BMI feature data.

In step C5, the obtained time interval feature data, optical feature sequences, metabolic heat feature sequences and physiological feature sequences are output as processing results.

Fig. 2 is a modular structure diagram of a blood glucose prediction device combining a big data model and a personalized model according to Embodiment 2 of the present invention. The device can be terminal equipments or a server for implementing the method according to the embodiment of the present invention, or a device connected with the terminal equipment or the server for implementing the method according to the embodiment of the invention. For example, the device can be a device or a chip system of the terminal equipment or the server. As shown in Fig. 2, the device comprises a big data model preparation module 201, a data receiving module 202, a personalized model preparation module 203 and a blood glucose prediction processing module 204.

The big data model preparation module 201 is configured to train a big data blood glucose prediction model based on a preset big data base and confirm a big data calibrated record.

The data receiving module 202 is configured to receive a first collection data set of a specified object, the first collection data set comprising a first data type, and the first data type being a prediction type or a label type.

The personalized model preparation module 203 is configured to, when the first data type is a label type, update a personalized data base corresponding to the specified object according to the first collection data set; if the data base is successfully updated, count the total number of personalized data records of the personalized data base to generate a corresponding first total number; if the first total number is greater than or equal to a preset first threshold value, perform screening optimal personalized data calibrated record in the personalized data base based on the big data blood glucose prediction model to obtain a latest personalized data calibrated record; if the first total number is equal to a preset second threshold value, train a personalized blood glucose prediction model based on the big data blood glucose prediction model, the personalized data base and the personalized data calibrated record; the first threshold value being smaller than the second threshold value.

The blood glucose prediction processing module 204 is configured to, when the first data type is a prediction type, count the total number of personalized data records in the personalized data base to generate a corresponding second total number; if the second total number is smaller than the second threshold value, perform blood glucose prediction processing on the first collection data set based on the second total number, the big data calibrated record, the personalized data calibrated record and the big data blood glucose prediction model to generate corresponding first predicted blood glucose data; and if the second total number is equal to the second threshold value, perform personalized blood glucose prediction processing on the first collection data set based on the personalized data calibrated record, the big data blood glucose prediction model and the personalized blood glucose prediction model to generate corresponding second predicted blood glucose data.

The blood glucose prediction device combining the big data model and the personalized model provided by the embodiment of the present invention can implement the method steps in the above method embodiments, and implementation principles and technical effects are similar, which will not be repeated here.

It should be understood that the division of different modules of the above device is based on logical functions, and in actual implementation, all or part of the modules can be integrated into a physical entity, or they can be physically separated. These modules can all be implemented in the form of software which can be called by processing elements, or all of them can be implemented in the form of hardware, or some modules are implemented in the form of software which can be called by processing elements, and some modules are implemented in the form of hardware. For example, the big data model preparation module may be a separate processing element, or may be integrated into a certain chip of the above-mentioned device, or it may be stored in a memory of the above-mentioned device in the form of program code, and called by a certain processing element of the above-mentioned device to implement the functions of the above-mentioned determination module. Other modules are implemented similarly. In addition, all or part of these modules may be integrated or implemented separately. The processing element described here may be an integrated circuit with signal processing capability. In the implementation process, each step of the above method or each module may be realized by an integrated logic circuit of hardware in the processor element or instructions in the form of software.

For example, the above modules may be one or more integrated circuits configured to implement the above method, such as one or more application specific integrated circuits (ASIC), one or more digital signal processors (DSP), one or more field programmable gate arrays (FPGA), etc. For another example, when one of the above modules is implemented in the form of a program code which can be called by a processing element, the processing element may be a general purpose processor, such as a central processing unit (CPU) or other processors which can call the program code. For example, these modules can be integrated and implemented in the form of system-on-a-chip (SOC).

The above embodiments can be realized fully or partially by software, hardware, firmware or any combination thereof. When implemented by software, it can be fully or partially implemented in the form of a computer program product. The computer program product comprises one or more computer instructions. When the computer program instructions are loaded and executed on a computer, all or part of the processes or functions described in the embodiments of the present invention are generated. The computer may be a general-purpose computer, a special-purpose computer, a computer network, or other programmable devices. The above computer instructions may be stored in a computer-readable storage medium or transmitted from one computer-readable storage medium to another. For example, the above computer instructions may be transmitted from one website, computer, server or data center to another website, computer, server or data center by wired (such as coaxial cable, optical fiber, digital subscriber line (DSL)) or wireless (such as infrared, wireless, Bluetooth, microwave) means. The computer-readable storage medium may be any available medium that a computer can access or a data storage device such as a server or a data center that contains one or more available media. The available media may be magnetic media (such as floppy disk, hard disk, magnetic tape), optical media (such as DVD), or semiconductor media (such as solid state disk (SSD)) and the like.

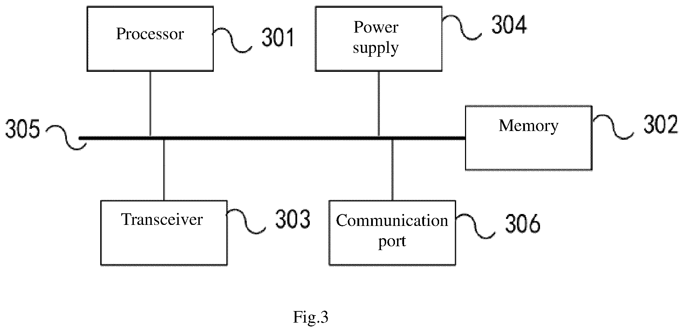

Fig. 3 is a structural diagram of electronic equipment according to Embodiment 3 of the present invention. The electronic equipment may be the aforementioned terminal equipment or server, or may be terminal equipment or server connected to the aforementioned terminal equipment or server to realize the method of the embodiment of the present invention. As shown in Fig. 3, the electronic equipment may comprise a processor 301 (for example, CPU), a memory 302, and a transceiver 303. The transceiver 303 is coupled to the processor 301, and the processor 301 controls the transceiving action of the transceiver 303. The memory 302 may store various instructions for accomplishing various processing functions and realizing the method and processing procedures provided in the above embodiments of the present invention. Preferably, the electronic equipment according to the embodiment of the present invention further comprises a power supply 304, a system bus 305 and a communication port 306. The system bus 305 is configured to realize communication between components. The communication port 306 is used for communication between the electronic equipment and other peripherals.

The system bus mentioned in Fig. 3 may be a peripheral component interconnect (PCI) bus or an extended industry standard architecture (EISA) bus, etc. The system bus may be divided into an address bus, a data bus and a control bus. For convenience of representation, only a thick line is shown in the figure, but it does not mean that there is only one bus or one type of bus. The communication interface is configured to realize communication between a data base access device and other devices (such as client, read-write library and read-only library). The memory may comprise a random access memory (RAM) or a non-volatile memory, such as at least one disk memory.

The above processor may be a general-purpose processor, like a central processing unit (CPU) and a network processor (NP). It may also be a digital signal processor (DSP), an application specific integrated circuit (ASIC), a field programmable gate array (FPGA) or other programmable logic devices, discrete gate or transistor logic devices, and discrete hardware components.

It should be noted that an embodiment of the present invention also provides a computer-readable storage medium, in which instructions are stored, which, when run on a computer, cause the computer to execute the method and process provided in the above embodiment.

An embodiment of the present invention also provides a chip for running instructions. The chip is used for executing the method and process provided in the above embodiment.

The embodiments of the present invention provide a blood glucose prediction method and device combining a big data model and a personalized model, electronic equipment and a computer-readable storage medium. A big data blood glucose prediction model reflecting the correlation between optics, metabolic heat and blood glucose level changes is trained first, and a data record is selected from a big data base as a calibrated record of the model. A personalized data base is created for a specified object to store label data of a current object, that is, personalized records; after a sufficient number of personalized records are stored in the personalized data base, a personalized calibrated record more suitable for the specified object is selected from the personalized data base based on the well-trained big data blood glucose prediction model; and when the number of the personalized records reaches a specified threshold value, a personalized blood glucose prediction model is trained based on the well-trained big data blood glucose prediction model, the personalized calibrated record and the personalized data base. During blood glucose prediction of the specified object, if the total number of the personalized records has not reached the specified threshold value, the big data blood glucose prediction model is used for blood glucose prediction of the specified object; and if the total number of the personalized records reaches the specified threshold value, blood glucose prediction is performed on the specific object based on the big data blood glucose prediction model and the personalized blood glucose prediction model. By means of the present invention, in the case of insufficient precipitation of personalized data, blood glucose prediction is performed based on the big data blood glucose prediction model; and under the condition of sufficient personalized data precipitation, a corresponding personalized blood glucose prediction model is trained for each specified object, and personalized blood glucose prediction is performed for each object based on the big data blood glucose prediction model and the personalized blood glucose prediction model. In this way, not only can the inconvenience caused by invasive blood glucose tests be solved, but also different prediction model schemes can be provided for different objects, thus achieving the purposes of reducing observation difficulty and improving the level of prediction customization.

Those skilled in the art should further realize that the units and algorithm steps of each example described in connection with the embodiments disclosed herein can be implemented in electronic hardware, computer software or a combination of the two. In order to clearly explain the interchangeability of hardware and software, the components and steps of each example have been generally described according to functions in the above description. Whether these functions are implemented by hardware or software depends on the specific application and design constraints of the technical scheme. Those skilled in the art can use different methods to realize the described functions for each specific application, but this implementation should not be considered beyond the scope of the invention.

The steps of a method or algorithm described in connection with the embodiments disclosed herein may be implemented by hardware, a software module executed by a processor, or a combination of the two. The software module may be placed in a random access memory (RAM), internal memory, read-only memory (ROM), electrically programmable ROM, electrically erasable programmable ROM, register, hard disk, removable disk, CD-ROM, or any other storage medium known in the technical field.

The above-mentioned specific embodiments further explain the purpose, technical solution and beneficial effects of the present invention in detail. It should be understood that the above are only specific embodiments of the present invention and are not used to limit the scope of protection of the present invention. Any modification, equivalent substitution, improvement, etc. made within the spirit and principles of the present invention should be included in the scope of protection of the present invention.

## Claims

1. A blood glucose prediction method combining a big data model and a personalized model, comprising:
training a big data blood glucose prediction model based on a preset big data base and confirming a big data calibrated record;
receiving a first collection data set of a specified object, the first collection data set comprising a first data type, and the first data type being a prediction type or a label type;
when the first data type is a label type, updating a personalized data base corresponding to the specified object according to the first collection data set; if the data base is successfully updated, counting a total number of personalized data records of the personalized data base to generate a corresponding first total number; if the first total number is greater than or equal to a preset first threshold value, performing optimal personalized data calibrated record screening in the personalized data base based on the big data blood glucose prediction model to obtain a latest personalized data calibrated record; if the first total number is equal to a preset second threshold value, training a personalized blood glucose prediction model based on the big data blood glucose prediction model, the personalized data base and the personalized data calibrated record; the first threshold value being smaller than the second threshold value;
when the first data type is a prediction type, counting the total number of personalized data records in the personalized data base to generate a corresponding second total number; if the second total number is smaller than the second threshold value, performing blood glucose prediction processing on the first collection data set based on the second total number, the big data calibrated record, the personalized data calibrated record and the big data blood glucose prediction model to generate corresponding first predicted blood glucose data; and if the second total number is equal to the second threshold value, performing personalized blood glucose prediction processing on the first collection data set based on the personalized data calibrated record, the big data blood glucose prediction model and the personalized blood glucose prediction model to generate corresponding second predicted blood glucose data.

2. The blood glucose prediction method combining the big data model and the personalized model according to claim 1, wherein
a neural network of the big data blood glucose prediction model adopts a fully connected network structure; and
a model structure of the personalized blood glucose prediction model is a support vector machine model structure, a random forest model structure or a decision tree model structure.

3. The blood glucose prediction method combining the big data model and the personalized model according to claim 1, wherein
the big data base comprises a plurality of big data records; the big data records comprise first preprandial sub-records and first postprandial sub-records; the first preprandial sub-record comprises a first collection time, a first time interval, a first optical data group, a first metabolic heat data group, a first physiological information data group and a first measured blood glucose level; the first postprandial sub-record comprises a second collection time, a second time interval, a second optical data group, a second metabolic heat data group, a second physiological information data group and a second measured blood glucose level; the first and second measured blood glucose levels correspond to preprandial and postprandial measured blood glucose levels respectively;
when the first data type is a label type, the first collection data set further comprises a first preprandial collection record and a first postprandial collection record; the first preprandial collection record comprises a third collection time, a third time interval, a third optical data group, a third metabolic heat data group, a third physiological information data group and a third measured blood glucose level; the first postprandial collection record comprises a fourth collection time, a fourth time interval, a fourth optical data group, a fourth metabolic heat data group, a fourth physiological information data group and a fourth measured blood glucose level; the third and fourth measured blood glucose levels correspond to preprandial and postprandial measured blood glucose levels respectively;
when the first data type is a prediction type, the first collection data set further comprises a fifth collection time, a fifth time interval, a fifth optical data group, a fifth metabolic heat data group and a fifth physiological information data group;
the personalized data base comprises the plurality of personalized data records; the personalized data records comprise second preprandial sub-records and second postprandial sub-records; the second preprandial sub-record comprises a sixth collection time, a sixth time interval, a sixth optical data group, a sixth metabolic heat data group, a sixth physiological information data group and a fifth measured blood glucose level; the second postprandial sub-record comprises a seventh collection time, a seventh time interval, a seventh optical data group, a seventh metabolic heat data group, a seventh physiological information data group and a sixth measured blood glucose level; the fifth and sixth measured blood glucose levels correspond to preprandial and postprandial measured blood glucose levels respectively; and the maximum total number of records in the personalized data base is the second threshold value.

4. The blood glucose prediction method combining the big data model and the personalized model according to claim 3, wherein the training a big data blood glucose prediction model based on a preset big data base and confirming a big data calibrated record comprises:
extracting a plurality of big data records from the big data base to form a big data set;
performing first feature vector conversion processing on each of the first preprandial sub-records or the first postprandial sub-records in the big data set to generate first feature vectors corresponding to each sub-record; recording the first measured blood glucose levels or the second measured blood glucose levels corresponding to each of the first feature vectors as corresponding first feature blood glucose levels;
selecting a big data record from the big data set as the big data calibrated record; taking two first feature vectors of the big data calibrated record as a corresponding big data preprandial calibration vector and a big data postprandial calibration vector respectively, and taking two first feature blood glucose levels as a corresponding big data preprandial calibrated blood glucose level and a big data postprandial calibrated blood glucose level respectively;
recording all the other first feature vectors except the big data preprandial calibration vector and the big data postprandial calibration vector as second feature vectors; forming corresponding first model training vectors by each of the second feature vectors, the big data preprandial calibration vector, the big data postprandial calibration vector, the big data preprandial calibrated blood glucose level and the big data postprandial calibrated blood glucose level;
based on the big data blood glucose prediction model, performing big data model prediction processing on any of the first model training vectors to obtain corresponding first predicted data, and generating corresponding current predicted blood glucose data according to the sum of the big data postprandial calibrated blood glucose level and the first predicted data; calculating a training error between the current predicted blood glucose data and the first feature blood glucose level corresponding to the current first model training vector using a loss function of the big data blood glucose prediction model; when the training error does not meet a preset reasonable error range, modulating the big data blood glucose prediction model based on the training error, and selecting the next first model training vector to continue training the modulated big data blood glucose prediction model; and stopping training when the training error meets the reasonable error range.

5. The blood glucose prediction method combining the big data model and the personalized model according to claim 3, wherein the step of updating a personalized data base corresponding to the specified object according to the first collection data set comprises:
counting the total number of personalized data records in the personalized data base to generate a corresponding current total number; when the current total number is smaller than the second threshold value, adding a new personalized data record as a current record in the personalized data base; when the current total number is equal to the second threshold value, taking the personalized data record with the earliest addition time in the personalized data base as the current record; and
extracting the first preprandial collection record from the first collection data set as the second preprandial sub-record of the current record and storing the same in the personalized data base, and extracting the first postprandial collection record as the second postprandial sub-record of the current record and storing the same in the personalized data base.

6. The blood glucose prediction method combining the big data model and the personalized model according to claim 3, wherein the step of performing optimal personalized data calibrated record screening in the personalized data base based on the big data blood glucose prediction model to obtain a latest personalized data calibrated record comprises:
performing first feature vector conversion processing on the second preprandial sub-record or the second postprandial sub-record of each of the personalized data records in the personalized data base to generate third feature vectors corresponding to each sub-record; recording the fifth measured blood glucose levels or the sixth measured blood glucose levels corresponding to each of the third feature vectors as corresponding second feature blood glucose levels;
sequentially extracting the personalized data records as a current calibrated record in the personalized data base; taking two third feature vectors of the current calibrated record as a corresponding big data preprandial calibration vector and a big data postprandial calibration vector respectively, and taking two second feature blood glucose levels as a corresponding big data preprandial calibrated blood glucose level and a big data postprandial calibrated blood glucose level respectively; recording third feature vectors of other personalized data records except the current calibrated record as fourth feature vectors, and forming corresponding first model input vectors by each of the fourth feature vectors, the big data preprandial calibration vector, the big data postprandial calibration vector, the big data preprandial calibrated blood glucose level and the big data postprandial calibrated blood glucose level; based on the big data blood glucose prediction model, performing big data model prediction processing on each of the first model input vectors to obtain corresponding second predicted data, and generating corresponding first model predicted blood glucose data according to the sum of the big data postprandial calibrated blood glucose level and the second predicted data; calculating an absolute difference between each of the first model predicted blood glucose data and the second feature blood glucose level corresponding to a current first model input vector to obtain a corresponding first prediction error; calculating the sum of all the obtained first prediction errors to obtain a first prediction error sum corresponding to the current calibrated record; and
taking a minimum prediction error sum as an optimal calibration personalized record group screening principle, and taking the personalized data record with the minimum first prediction error sum in the personalized data base as the latest personalized data calibrated record.

7. The blood glucose prediction method combining the big data model and the personalized model according to claim 3, wherein the step of training a personalized blood glucose prediction model based on the big data blood glucose prediction model, the personalized data base and the personalized data calibrated record comprises:
performing first feature vector conversion processing on the second preprandial sub-record or the second postprandial sub-record of each of the personalized data records in the personalized data base to generate fifth feature vectors corresponding to each sub-record; recording the fifth measured blood glucose levels or the sixth measured blood glucose levels corresponding to each of the fifth feature vectors as corresponding third feature blood glucose levels;
taking two fifth feature vectors of the personalized data calibrated record as a corresponding big data preprandial calibration vector and a big data postprandial calibration vector respectively, and taking two third feature blood glucose levels as a corresponding big data preprandial calibrated blood glucose level and a big data postprandial calibrated blood glucose level respectively;
recording the fifth feature vectors of other personalized data records except the personalized data calibrated record in the personalized data base as sixth feature vectors; forming corresponding second model training vectors by each of the sixth feature vectors, the big data preprandial calibration vector, the big data postprandial calibration vector, the big data preprandial calibrated blood glucose level and the big data postprandial calibrated blood glucose level;
performing big data model prediction processing on each of the second model training vectors based on the big data blood glucose prediction model to obtain corresponding third predicted data, and generating corresponding big data predicted blood glucose data according to the sum of the big data postprandial calibrated blood glucose level and the third predicted data;
according to a preset big data-personalized feature association status, extracting part of feature data from the sixth feature vectors corresponding to each of the second model training vectors to form corresponding seventh feature vectors, extracting part of feature data from the big data preprandial calibration vector to form a corresponding personalized preprandial calibration vector, and extracting part of feature data from the big data postprandial calibration vector to form a corresponding personalized postprandial calibration vector; forming corresponding third model training vectors by the seventh feature vectors, the personalized preprandial calibration vector, the personalized postprandial calibration vector, the big data preprandial calibrated blood glucose level, the big data postprandial calibrated blood glucose level and the big data predicted blood glucose data;
performing personalized model prediction processing on any of the third model training vectors based on the personalized blood glucose prediction model to obtain corresponding fourth predicted data, and generating corresponding current predicted blood glucose data according to the sum of the big data predicted blood glucose data and the fourth predicted data; calculating an absolute difference between the current predicted blood glucose data and the third feature blood glucose level corresponding to a current third model training vector as a current measurement error; modulating the personalized blood glucose prediction model when the current measurement error does not match a preset reasonable error range; and then selecting the next third model training vector to train the personalized blood glucose prediction model until training with the last third model training vector is completed.

8. The blood glucose prediction method combining the big data model and the personalized model according to claim 7, wherein the step of, according to a preset big data-personalized feature association status, extracting part of feature data from the sixth feature vectors corresponding to each of the second model training vectors to form corresponding seventh feature vectors, extracting part of feature data from the big data preprandial calibration vector to form a corresponding personalized preprandial calibration vector, and extracting part of feature data from the big data postprandial calibration vector to form a corresponding personalized postprandial calibration vector, comprises:
identifying whether each status bit of the big data-personalized feature association status is an association status, and if so, adding matching feature data of each status bit in the sixth feature vectors, the big data preprandial calibration vector and the big data postprandial calibration vector to the corresponding seventh feature vectors, the personalized preprandial calibration vector and the personalized postprandial calibration vector, the big data-personalized feature association status comprising first, second, third and fourth status bits, the matching feature data of the first status bit being time interval feature data, the matching feature data of the second status bit being optical feature sequences, the matching feature data of the third status bit being metabolic heat feature sequences, and the matching feature data of the fourth status bit being physiological feature sequences.

9. The blood glucose prediction method combining the big data model and the personalized model according to claim 3, wherein the step of performing blood glucose prediction processing on the first collection data set based on the second total number, the big data calibrated record, the personalized data calibrated record and the big data blood glucose prediction model to generate corresponding first predicted blood glucose data comprises:
determining whether the second total number is smaller than the first threshold value; if the second total number is smaller than the first threshold value, performing first feature vector conversion processing on the first preprandial sub-record and the first postprandial sub-record of the big data calibrated record, taking two obtained feature vectors as a corresponding big data preprandial calibration vector and a big data postprandial calibration vector, and taking the first measured blood glucose level and the second measured blood glucose level of the big data calibrated record as a corresponding big data preprandial calibrated blood glucose level and a big data postprandial calibrated blood glucose level; if the second total number is greater than or equal to the first threshold value, performing first feature vector conversion processing on the second preprandial sub-record and the second postprandial sub-record of the personalized data calibrated record, taking two obtained feature vectors as a corresponding big data preprandial calibration vector and a big data postprandial calibration vector, and taking the fifth measured blood glucose level and the sixth measured blood glucose level of the personalized data calibrated record as the corresponding big data preprandial calibrated blood glucose level and the big data postprandial calibrated blood glucose level;
performing first feature vector conversion processing on the first collection data set to generate corresponding eighth feature vectors; forming corresponding second model input vectors by the eighth feature vectors, the big data preprandial calibration vector, the big data postprandial calibration vector, the big data preprandial calibrated blood glucose level and the big data postprandial calibrated blood glucose level;
performing big data model prediction processing on the second model input vectors based on the big data blood glucose prediction model to obtain corresponding fifth predicted data; and generating the corresponding first predicted blood glucose data according to the sum of the big data postprandial calibrated blood glucose level and the fifth predicted data.

10. The blood glucose prediction method combining the big data model and the personalized model according to claim 3, wherein the step of performing personalized blood glucose prediction processing on the first collection data set based on the personalized data calibrated record, the big data blood glucose prediction model and the personalized blood glucose prediction model to generate corresponding second predicted blood glucose data comprises:
performing first feature vector conversion processing on the second preprandial sub-record and the second postprandial sub-record of the personalized data calibrated record to obtain two ninth feature vectors, taking the two ninth feature vectors as a corresponding big data preprandial calibration vector and a big data postprandial calibration vector respectively, and taking the fifth and sixth measured blood glucose levels of the personalized data calibrated record as a corresponding big data preprandial calibrated blood glucose level and a big data postprandial calibrated blood glucose level respectively;
performing first feature vector conversion processing on the first collection data set to generate corresponding tenth feature vectors; forming corresponding third model input vectors by the tenth feature vectors, the big data preprandial calibration vector, the big data postprandial calibration vector, the big data preprandial calibrated blood glucose level and the big data postprandial calibrated blood glucose level;
performing big data model prediction processing on the third model input vectors based on the big data blood glucose prediction model to obtain corresponding sixth predicted data; generating corresponding big data predicted blood glucose data according to the sum of the big data postprandial calibrated blood glucose level and the sixth predicted data;
according to a preset big data-personalized feature association status, extracting part of feature data from the tenth feature vectors to form corresponding eleventh feature vectors, extracting part of feature data from the big data preprandial calibration vector to form a corresponding personalized preprandial calibration vector, and extracting part of feature data from the big data postprandial calibration vector to form a corresponding personalized postprandial calibration vector;
forming corresponding fourth model input vectors by the eleventh feature vectors, the personalized preprandial calibration vector, the personalized postprandial calibration vector, the big data preprandial calibrated blood glucose level, the big data postprandial calibrated blood glucose level and the big data predicted blood glucose data;
performing personalized model prediction processing on the fourth model input vectors based on the personalized blood glucose prediction model to obtain corresponding seventh predicted data; and generating the corresponding second predicted blood glucose data according to the sum of the big data predicted blood glucose data and the seventh predicted data.

11. A device for realizing the steps of the blood glucose prediction method combining the big data model and the personalized model according to any one of claims 1-10, comprising a big data model preparation module, a data receiving module, a personalized model preparation module and a blood glucose prediction processing module, wherein
the big data model preparation module is configured to train a big data blood glucose prediction model based on a preset big data base and confirm a big data calibrated record;
the data receiving module is configured to receive a first collection data set of a specified object, the first collection data set comprising a first data type, and the first data type being a prediction type or a label type;
the personalized model preparation module is configured to, when the first data type is a label type, update a personalized data base corresponding to the specified object according to the first collection data set; if the data base is successfully updated, count a total number of personalized data records of the personalized data base to generate a corresponding first total number; if the first total number is greater than or equal to a preset first threshold value, perform optimal personalized data calibrated record screening in the personalized data base based on the big data blood glucose prediction model to obtain a latest personalized data calibrated record; if the first total number is equal to a preset second threshold value, train a personalized blood glucose prediction model based on the big data blood glucose prediction model, the personalized data base and the personalized data calibrated record; the first threshold value being smaller than the second threshold value; and
the blood glucose prediction processing module is configured to, when the first data type is a prediction type, count the total number of personalized data records in the personalized data base to generate a corresponding second total number; if the second total number is smaller than the second threshold value, perform blood glucose prediction processing on the first collection data set based on the second total number, the big data calibrated record, the personalized data calibrated record and the big data blood glucose prediction model to generate corresponding first predicted blood glucose data; and if the second total number is equal to the second threshold value, perform personalized blood glucose prediction processing on the first collection data set based on the personalized data calibrated record, the big data blood glucose prediction model and the personalized blood glucose prediction model to generate corresponding second predicted blood glucose data.

12. Electronic equipments, comprising a memory, a processor and a transceiver,
wherein the processor is configured to be coupled with the memory, and read and execute instructions in the memory, so as to realize the method according to any one of claims 1-10,
the transceiver is coupled with the processor, and the processor controls the transceiver to send and receive messages.

13. A computer-readable storage medium, which stores computer instructions that, when executed by a computer, cause the computer to execute the method according to any one of claims 1-10.
